# EUROPEAN PATENT APPLICATION

(11) **EP 4 495 245 A1**
(43) Date of publication of application: **22.01.2025**
(21) Application number: 23770934.0
(22) Date of filing: 14.03.2023
(51) Int. Cl.: C12N 15/85, A01K 67/027, C12N 5/0735, C12N 5/10, C12N 15/09, C12N 15/12

(54) **MHC GENE GROUP HUMANIZED ANIMAL**

(30) Priority: 14.03.2022 JP 2022039574
(71) Applicant: Tokyo Metropolitan Institute of Medical Science, Tokyo 156-8506 (JP)
(72) Inventor: SUZUKI, Teruhiko, Tokyo 156-8506 (JP); HARA, Takahiko, Tokyo 156-8506 (JP)
(74) Representative: J A Kemp LLP
(86) International application number: PCT/JP2023/010779
(87) International publication number: WO 2023/176982

(57) **Abstract**

A vector set including a combination of the following vectors (A), (B), and (D) or (C) and (D): (A) a targeting vector 1 incorporated at any position P1 distal to a human MHC region, (B) a targeting vector 2 incorporated at a position p1 corresponding to the P1 of a non-human MHC region, (C) a targeting vector 3 that triggers a recombination between a region from the P1 to the distal end and a region from the p1 to the distal end, and (D) a targeting vector 4 that triggers in the chromosome after recombination a recombination between a region from any position P2 proximal to a human MHC region to the distal end and a region from a position p2 corresponding to the P2 in a chromosome in which a non-human MHC is present to the distal end.

## Description

### [TECHNICAL FIELD]

The present invention relates to a non-human mammal with humanized major histocompatibility complex (MHC), and to a method for producing the same.

### [BACKGROUND ART]

Acquired immunity is an immune system in which three factors, MHC, T cell receptors, and antibodies, function in a coordinated manner to activate T cells and B cells to eliminate pathogens, virus-infected cells, cancer cells, and the like. Since acquired immunity can target specific molecules, it is used in the development of biopharmaceuticals. Antibody drugs are a typical example of such biopharmaceuticals, which are used to treat various diseases such as cancer, and their market size is said to be nearly 18 trillion yen by 2020 (Non-patent literature 1 and 2).

However, since antibodies can only recognize extracellular antigens, the problem of depletion of promising target molecules has been pointed out. Therefore, the development of medical and pharmaceutical products using T-cell receptors (TCRs) is currently receiving increased attention (Non-patent literature 3). Since TCRs can target intracellular proteins as well, a very large number of molecules can be targeted. Although many medical and pharmaceutical products using TCRs are already undergoing clinical trials, drug creation using TCRs itself has problems, and one of them is the lack of a suitable screening system using living bodies.

Because of the interspecies diversity of the three factors, i.e., MHC, TCRs, and antibodies, which play major roles in acquired immunity, it is difficult to create pharmaceutical products using acquired immunity in model animals. Recently, mice in which these genes are humanized have been generated and used for the development of pharmaceutical products (Non-patent literature 4-8), but only humanization of the MHC gene group has not yet been achieved. If MHC is of mouse origin, humanization of only TCRs will result in a mouse MHC-restricted TCRs, and thus it is difficult to screen TCRs adequately using mice.

The reason why humanization of only the MHC gene group has not been realized is that this humanization is technically very difficult. There are 21 MHC genes in humans and 39 in mice, and these genes are intermingled with various genes in a genomic region called the MHC region, which has about 3-4 million base pairs.

Bacterial artificial chromosomes (BACs) have been utilized as vectors that can accommodate large sequences for genome sequencing and the like, but even with a BAC, only about 200,000 base pairs can be accommodated, making it extremely difficult to introduce the entire MHC region using conventional transgenic techniques. A method of introducing cDNAs of a human MHC gene group instead of the human MHC region may be possible, but in this case, transgenic mice of each human MHC gene needs to be generated to generate mice expressing all human MHC genes by breeding. Since it takes at least about 6 months to establish a single transgenic mouse line and make it ready for breeding, it is practically very difficult to produce MHC gene group-humanized mice using conventional genetic engineering techniques.

For this reason, when mice expressing HLA are required, transgenic mice transfected only with a specific human MHC are used (Non-patent literature 9 and 10). However, since expression of each MHC gene is regulated according to the environment, introduction of cDNAs that do not contain the expression regulatory regions around the gene loci cannot accurately reproduce the human immune response.

### [CITATION LIST]

### [Non-Patent Literature]

[Non-patent literature 1] Goydel, R.S. and Rader, C. (2021) Antibody-based cancer therapy. Oncogene, 40, 3655-3664.
[Non-patent literature 2] Zahavi, D. and Weiner, L. (2020) Monoclonal Antibodies in Cancer Therapy. Antibodies (Basel), 9.
[Non-patent literature 3] Zhao, L. and Cao, Y. J. (2019) Engineered T Cell Therapy for Cancer in the Clinic. Front Immunol, 10, 2250.
[Non-patent literature 4] Tomizuka, K., Shinohara, T., Yoshida, H., Uejima, H., Ohguma, A., Tanaka, S., Sato, K., Oshimura, M. and Ishida, I. (2000) Double trans-chromosomic mice: maintenance of two individual human chromosome fragments containing Ig heavy and kappa loci and expression of fully human antibodies. Proc Natl Acad Sci U S A, 97, 722-727.
[Non-patent literature 5] Tomizuka, K., Yoshida, H., Uejima, H., Kugoh, H., Sato, K., Ohguma, A., Hayasaka, M., Hanaoka, K., Oshimura, M. and Ishida, I. (1997) Functional expression and germline transmission of a human chromosome fragment in chimaeric mice. Nat Genet, 16, 133-143.
[Non-patent literature 6] Li, L.P., Lampert, J.C., Chen, X., Leitao, C., Popovic, J., Muller, W. and Blankenstein, T. (2010) Transgenic mice with a diverse human T cell antigen receptor repertoire. Nat Med, 16, 1029-1034.
[Non-patent literature 7] Obenaus, M., Leitao, C., Leisegang, M., Chen, X., Gavvovidis, I., van der Bruggen, P., Uckert, W., Schendel, D.J. and Blankenstein, T. (2015). Identification of human T-cell receptors with optimal affinity to cancer antigens using antigen-negative humanized mice. Nat Biotechnol, 33, 402-407.
[Non-patent literature 8] Poncette, L., Chen, X., Lorenz, F.K. and Blankenstein, T. (2019) Effective NY-ESO-1-specific MHC II-restricted T cell receptors from antigen-negative hosts enhance tumor regression. J Clin Invest, 129, 324-335.
[Non-patent literature 9] Shultz, L.D., Saito, Y., Najima, Y., Tanaka, S., Ochi, T., Tomizawa, M., Doi, T., Sone, A., Suzuki, N., Fujiwara, H. et al. (2010) Generation of functional human T-cell subsets with HLA-restricted immune responses in HLA class I expressing NOD/SCID/IL2r gamma (null) humanized mice. Proc Natl Acad Sci U S A, 107, 13022-13027.
[Non-patent literature 10] Suzuki, M., Takahashi, T., Katano, I., Ito, R., Ito, M., Harigae, H., Ishii, N. and Sugamura, K. (2012) Induction of human humoral immune responses in a novel HLA-DR-expressing transgenic NOD/Shi-scid/γcnull mouse. Int Immunol, 24, 243-252.

### [SUMMARY OF INVENTION]

### [Technical Problem]

When human and mouse MHC regions were compared, it was found that the MHC regions were large, but the gene compositions within the regions were maintained in both regions, except for the species-specific genes. Therefore, it seems to be possible to completely humanize MHC by replacing the entire MHC region of mouse with that of human. However, it is impossible to introduce such a huge sequence using a general-purpose gene vector system, and the MHC region cannot be replaced using conventional techniques. In view of the above, it has been desired to develop a targeting vector for replacing non-human mammalian MHC with human MHC, and a technique for replacing non-human mammalian MHC with human MHC using this targeting vector.

### [Solution to Problem]

After diligent study to solve the above problem, the present inventors succeeded in replacing non-human mammalian MHC with human MHC, thereby completing the present invention.

Thus, the present invention is as follows.
[1] A vector set comprising a combination of the following targeting vectors (A), (B) and (D), or a combination of the following targeting vectors (C) and (D):
   (A) a targeting vector 1 that is incorporated into human chromosome 6 at an arbitrary position P1 located on the distal side from a major histocompatibility complex (human MHC) region on the short arm of the human chromosome 6,
      wherein the targeting vector 1 causes translocational recombination between a region extending from the aforementioned P1 to the distal end and a region on a non-human mammalian chromosome, the region extending from position p1, which corresponds to the aforementioned P1 and is located on the distal side from a MHC (non-human MHC) region existing on the non-human mammalian chromosome, to the distal end, and the targeting vector 1 also contains a gene cassette 1 containing a recombinase recognition sequence, one partial sequence of a drug resistance gene 1 for drug selection, which is reconstituted upon the translocational recombination, and a drug resistance gene 2 for drug selection after targeting;
   (B) a targeting vector 2 that is incorporated into the non-human mammalian chromosome in which the MHC region exists, at position p1 that corresponds to the aforementioned P1 and that is located on the distal side from the MHC (non-human MHC) region,
      wherein the targeting vector 2 causes translocational recombination between the region extending from the aforementioned p1 to the distal end and the region extending from the aforementioned P1 to the distal end on the human chromosome 6, and the targeting vector 2 also contains a gene cassette 2 containing a recombinase recognition sequence, the other sequence of the drug resistance gene 1, and a drug resistance gene 3 for drug selection after targeting;
   (C) a targeting vector 3 that causes translocational recombination between a region on human chromosome 6, the region extending from an arbitrary position P1, which is located on the distal side from a human MHC region and targeted for cleavage by a genome editing tool, to the distal end and a region on a non-human mammalian chromosome in which MHC exists, the region extending from position p1, which corresponds to the aforementioned P1 and is located on the distal side from the non-human MHC region, to the distal end,
      wherein the targeting vector 3 contains a homologous sequence of the sequence of partial region R1a located between the aforementioned P1 and the human MHC region, a drug resistance gene 4 for drug selection after targeting, and a homologous sequence of the sequence of partial region R2b located on the distal side from the aforementioned p1, or the targeting vector 3 contains a homologous sequence of the sequence of partial region R1b located on the distal side from the aforementioned P1, a drug resistance gene 4 for drug selection after targeting, and a homologous sequence of the sequence of partial region R2a located between the aforementioned p1 and the non-human MHC region; and
   (D) a targeting vector 4 that causes translocational recombination between a region extending from an arbitrary position P2, which is located on the proximal side from the human MHC region and targeted for cleavage by a genome editing tool, to the distal end and a region on the non-human mammalian chromosome in which the non-human MHC exists, the region extending from position p2, which corresponds to the aforementioned P2 and located on the proximal side from the non-human MHC region, to the distal end,
      wherein the targeting vector 4 contains a homologous sequence of the sequence of partial region R3a located on the proximal side from the aforementioned P2, a drug resistance gene 5 for drug selection after targeting, and a homologous sequence of the sequence of partial region R4b located between the aforementioned p2 and the non-human MHC region, or the targeting vector 4 contains a homologous sequence of the sequence of partial region R3b located between the aforementioned p2 and the human MHC region, a drug resistance gene 5 for drug selection after targeting, and a homologous sequence of the sequence of partial region R4a located on the proximal side from the aforementioned p2.
[2] The vector set according to [1], further comprising the following vectors (E) and (F):
   (E) a genome editing tool vector 1 that cleaves human chromosome 6 at the arbitrary position P1; and
   (F) a genome editing tool vector 2 that cleaves the non-human chromosome at the arbitrary position p1.
[3] The vector set according to [1], further comprising the following vectors (G) and (H):
   (G) a genome editing tool vector 3 that cleaves human chromosome 6 at the arbitrary position P2; and
   (H) a genome editing tool vector 4 that cleaves the non-human chromosome at the arbitrary position p2.
[4] The vector set according to any one of [1]-[3], wherein the human MHC region comprises a class I region, a class II region, a class III region, or a combination of these regions.
[5] The vector set according to [4], wherein the class I region comprises at least one selected from the group consisting of HLA-A, HLA-B, HLA-C, HLA-E, HLA-F, HLA-G, MICA, and MICB.
[6] The vector set according to [4], wherein the class II region comprises at least one selected from the group consisting of HLA-DP, HLA-DQ, HLA-DR, HLA-DM, and HLA-DO.
[7] The vector set according to [4], wherein the region comprising the class I region is a region containing HLA-A, HLA-B, HLA-C, HLA-E, HLA-F, HLA-G, MICA, and MICB.
[8] The vector set according to [4], wherein the region comprising the class II region is a region containing HLA-DP, HLA-DQ, HLA-DR, HLA-DM, and HLA-DO.
[9] The vector set according to [4] in which the region comprising both class I and class II regions is a region between the DAXX locus and the MOG locus.
[10] The vector set according to any one of [1]-[3], wherein the arbitrary position P1 is a position between the MOG locus and the GABBR1 locus.
[11] The vector set according to any one of [1]-[3], wherein the arbitrary position P2 is a position between the KIFC1 locus and the DAXX locus.
[12] The vector set according to any one of [1]-[3], wherein the non-human mammal is a rodent.
[13] The vector set according to [12], wherein the rodent is a mouse.
[14] The vector set according to [13], wherein the arbitrary position p1 is a position between the Mog locus and the Gabbr1 locus.
[15] The vector set according to [13], wherein the arbitrary position p2 is a position between the Kifc1 locus and the Daxx locus.
[16] The vector set according to any one of [1]-[3], wherein the drug resistance gene 1 is neomycin-resistance gene, the drug resistance gene 2 is hygromycin-resistance gene, and/or the drug resistance gene 3 is puromycin-resistance gene.
[17] The vector set according to any one of [1]-[3], wherein the drug resistance gene 4 is neomycin-resistance gene and/or the drug resistance gene 5 is blasticidin-resistance gene.
[18] The vector set according to any one of [1]-[3], wherein, in the gene cassette 1, the recombinase recognition sequence is loxP, the drug resistance gene 1 is neomycin-resistance gene, and the drug resistance gene 2 is hygromycin-resistance gene.
[19] The vector set according to any one of [1]-[3], wherein the gene cassette 1 comprises a construct represented by the following formula (1):

   BGHpA-3'Neo-SA-loxP-EF1-Hyg-P2A-mRuby2-RGpA (1)

   (wherein, BGHpA represents a bovine growth hormone-derived poly A addition signal sequence, 3'Neo represents a part of the 3' sequence of neomycin-resistance gene, SA represents a splicing acceptor sequence, loxP represents a recombinase recognition sequence, EF1 represents human elongation factor 1α-derived promoter sequence, Hyg represents hygromycin-resistance gene, P2A represents porcine teschovirus-derived 2A sequence, mRuby2 represents red fluorescent gene, and RGpA represents rabbit β-globin-derived poly A addition signal sequence).
[20] The vector set according to any one of [1]-[3], wherein, in the gene cassette 2, the recombinase recognition sequence is loxP, the drug resistance gene 1 is neomycin-resistance gene, and the drug resistance gene 3 is puromycin-resistance gene.
[21] The vector set according to any one of [1]-[3], wherein the gene cassette 2 comprises a construct represented by the following formula (2):

   EF1-EGFP-P2A-5'Neo-SD-loxP-SA-T2A-Puro-RGpA (2)

   (wherein, EF1 represents human elongation factor 1α-derived promoter sequence, EGFP represents green fluorescent gene, P2A represents porcine teschovirus-derived A sequence, 5'Neo represents a part of the 5' sequence of neomycin-resistance gene, SD represents a splicing donor sequence, loxP represents a recombinase recognition sequence, SA represents a splicing acceptor sequence, T2A represents Thosea asigna virus-derived 2A sequence, Puro represents puromycin-resistance gene, and RGpA represents rabbit β-globin-derived poly A addition signal sequence).
[22] A non-human mammalian cell with a humanized MHC region, the cell comprising a chromosome in which a MHC region on the non-human mammalian chromosome has been replaced with a MHC region on human chromosome by the vector set according to any one of [1]-[21].
[23] The cell according to [22], wherein the cell is an ES cell.
[24] A non-human mammal, which is generated from the cell according to either one of [22] and [23] and in which the MHC region has been humanized.
[25] The non-human mammal according to [24], wherein the non-human mammal is a rodent.
[26] The non-human mammal according to [25], wherein the rodent is a mouse.
[27] The non-human mammal according to [26], comprising a chromosome in which a chromosome portion extending at least from the Daxx locus to the Mog locus on mouse chromosome 17 has been replaced with a chromosome portion extending at least from the DAXX locus to the MOG locus on human chromosome 6.
[28] A method for producing a non-human mammal with a humanized MHC region, the method comprising the steps of:
   (1) generating a cell comprising a chromosome in which a MHC region on a non-human mammalian chromosome has been replaced with a MHC region on human chromosome using the vector set according to any one of [1]-[21]; and
   (2) creating a non-human mammal with a humanized MHC region from the cell obtained in (1) above.
[29] The method according to [28], wherein the non-human mammal is a rodent.
[30] A method for producing a non-human mammal with a humanized MHC region, the method comprising the step of transplanting into a foster parent of the non-human mammal according to [29] wherein the rodent is a mouse to create a non-human mammal having a chromosome with a humanized MHC region.
[31] A method for producing a non-human mammal with a humanized MHC region, the method comprising the step of transplanting the cell according to [22] into a foster parent of a non-human mammal to create a non-human mammal having a chromosome with a humanized MHC region.
[32] The method according to [31], wherein the non-human mammal is a rodent.
[33] The method according to [32], wherein the rodent is a mouse.

### [ADVANTAGEOUS EFFECTS OF INVENTION]

The present invention has enabled the creation of a non-human animal having a chromosome in which the non-human MHC has been replaced with the human MHC. This will enable the use of non-human animals in the development of pharmaceutical products (e.g., tumor immunotherapeutics) and reagents that utilize the human immune system.

### [BRIEF DESCRIPTION OF DRAWINGS]

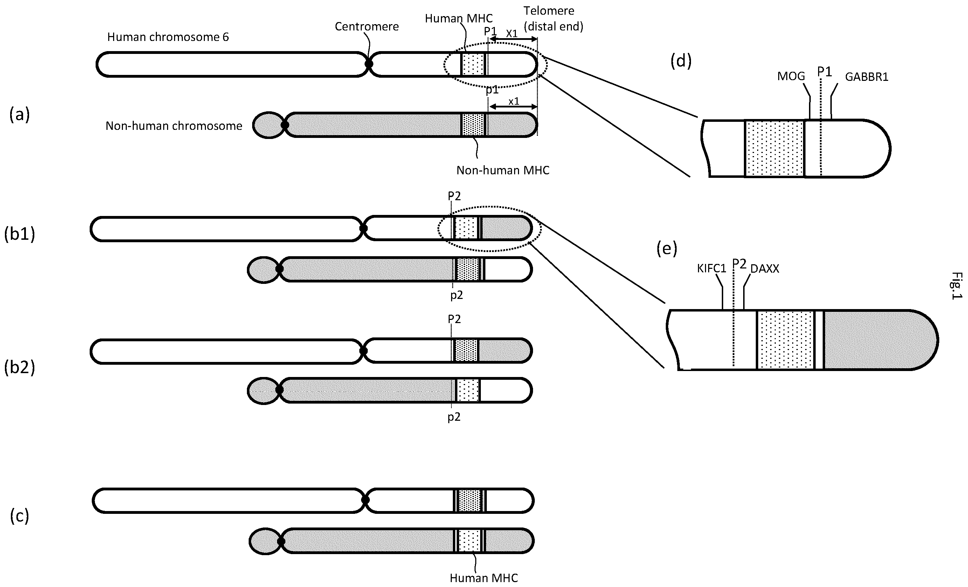

[Figure 1] Schematic views showing recombination for replacing non-human MHC with human MHC according to the present invention. The figure is a schematic view showing a case where the non-human animal is a mouse.

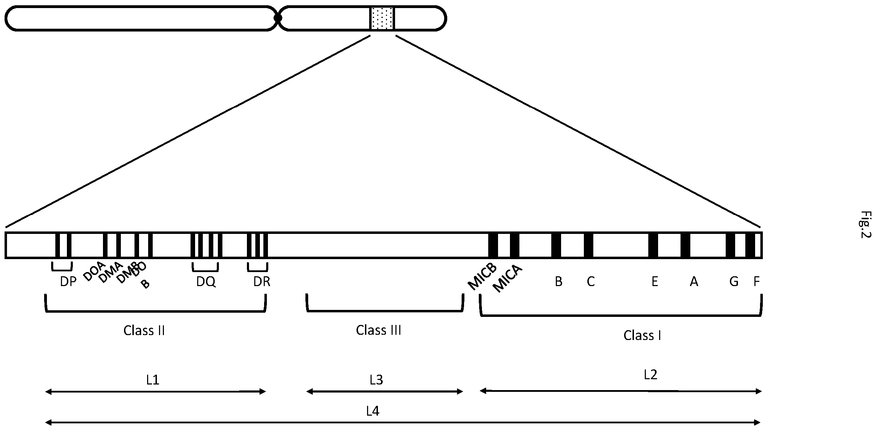

[Figure 2] A schematic view showing the MHC region in human chromosome.

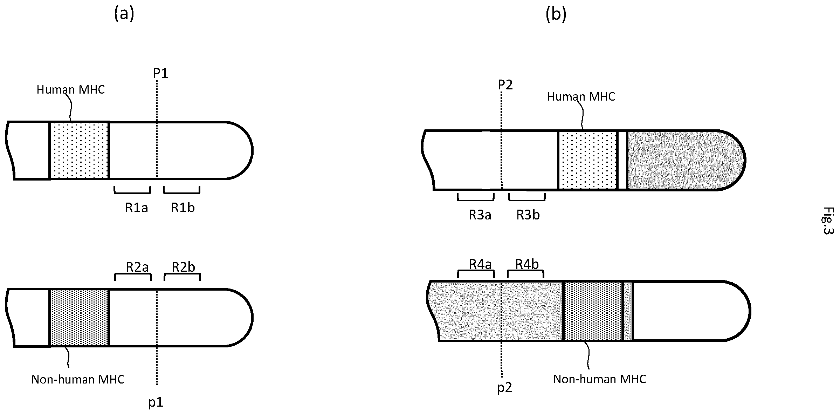

[Figure 3] A view showing the targeted regions when using genome editing targeting vectors.

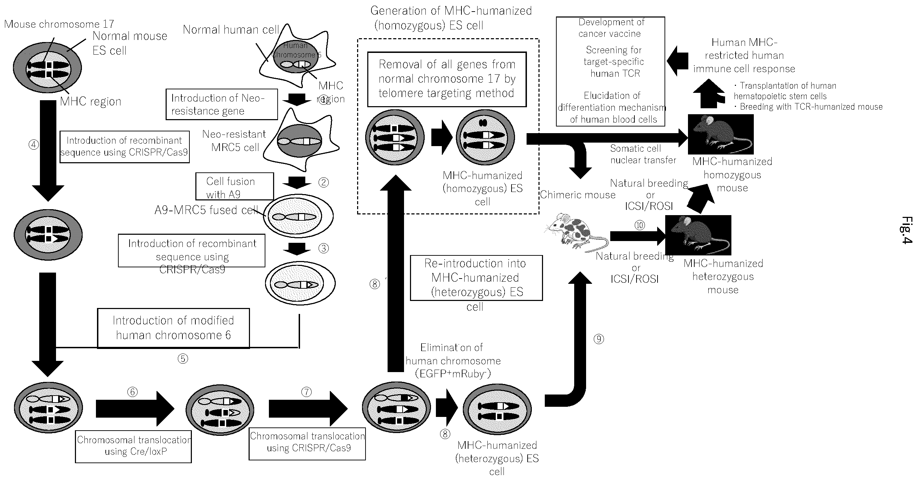

[Figure 4] A schematic view showing a process of generating a MHC region-humanized mouse.

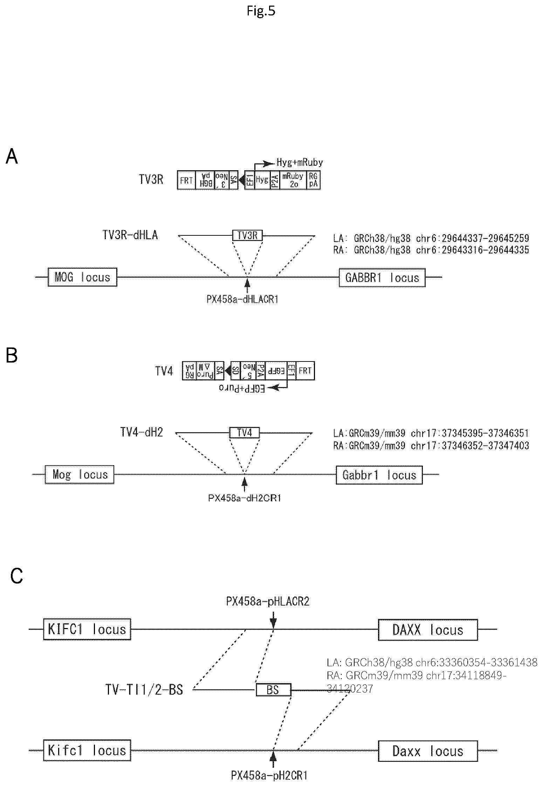

[Figure 5] Views showing structures of targeting vectors. (A) Structure of TV3R-dHLA. (B) Structure of TV4-dH2. (C) Structure of TV-Tl1/2-BS. The genomic locations of homology arms used for the targeting vectors are shown in the figure.

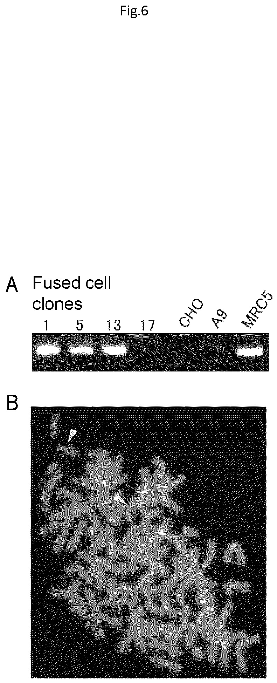

[Figure 6] Views showing the isolation of A9-MRC5 fused cell clones having human chromosome 6. (A) Genomic DNAs were prepared from the isolated A9-MRC5 fused cell clones (hChA9#1, 5, 13, 17) and analyzed by genomic PCR for the presence of a sequence on human chromosome 6. (B) The presence of human chromosome 6 in hChA9#5 was confirmed by FISH analysis. Red: Human chromosome 6 centromere-specific probe.

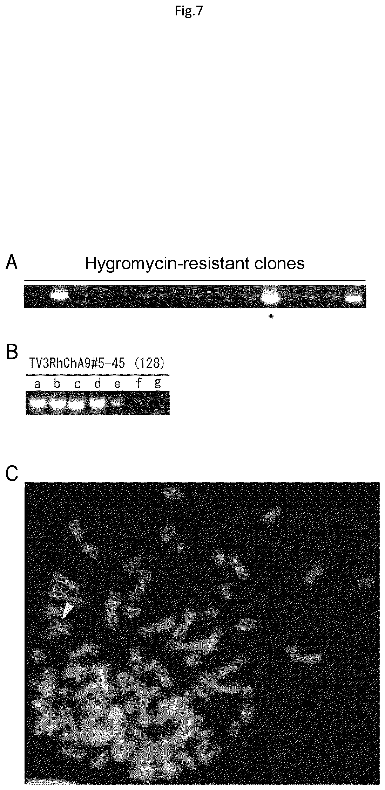

[Figure 7] Views showing targeting of TV3R. (A) Targeting of TV3R-dHLA was performed in A9-MRC5 fused cell clones, and hygromycin-resistant clones were isolated and analyzed by genomic PCR to see if recombination transfer occurred. * represents TV3RhChA9#5-45 (128) clone. (B) Recloning was performed with TV3RhChA9#5-45 (128) and clones were analyzed by genomic PCR. (C) TV3RhChA9#5-45a was analyzed by FISH. Red: Human chromosome 6 centromere-specific probe.

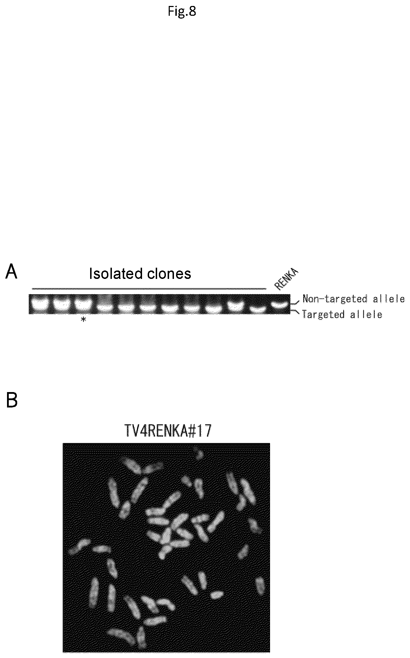

[Figure 8] Views showing targeting of TV4. (A) TV4-dH2 targeting was analyzed by genomic PCR. * represents TV4RENKA#17 clone. (B) DAPI staining confirmed that TV4RENKA#17 had 40 chromosomes.

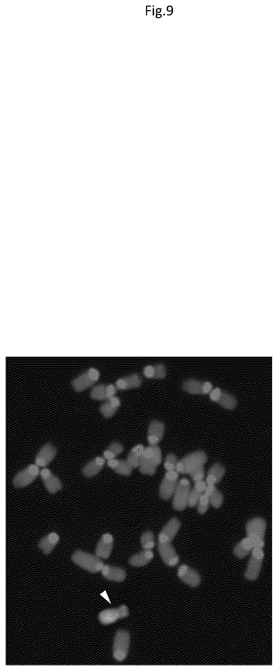

[Figure 9] Views showing the isolation of TV3-4RENKA clones. Modified human chromosome 6-transfected cell TV3-4RENKA#17/5-45a-3 was analyzed by FISH. The arrowhead indicates the introduced modified human chromosome 6. The number of chromosomes was confirmed to be 41. Red: Mouse Cot-1 (probe of mouse genome sequence), purple: RP11-54H13 (probe within the human MHC region).

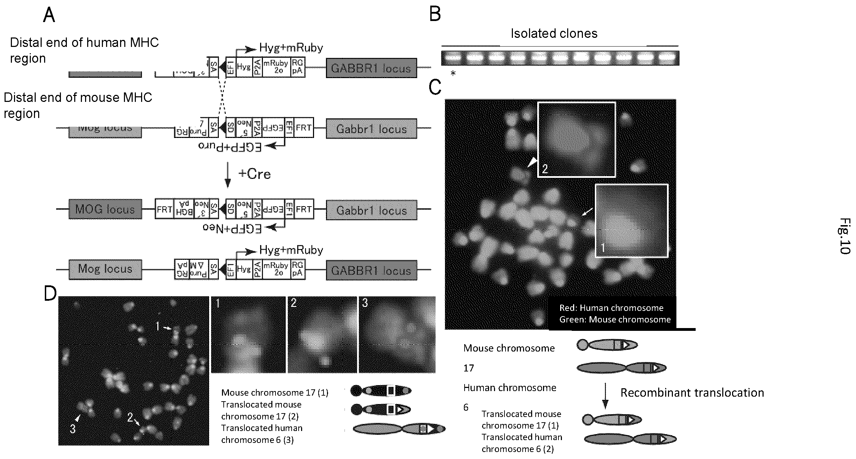

[Figure 10] Views showing the isolation of TV3-4RENKATl3/4 clones. (A) Recombinant translocation by Cre/loxP. (B) Confirmation of translocation by genomic PCR. * represents TV3-4RENKATl3/4#17-1. (C) TV3-4RENKATl3/4#17-1 was analyzed by FISH. The numbers shown in the FISH images correspond to the numbers assigned to the translocated chromosomes shown in the schematic view. Red: Human Cot-1 (probe of human genome sequence), Green: Mouse Cot-1 (probe of mouse genome sequence). (D) TV3-4RENKATl3/4#17-1 was analyzed by FISH. The numbers shown in the FISH images correspond to the numbers assigned to the translocated chromosomes shown in the schematic view. Red: RP11-54H13 (probe within the human MHC region), Green: RP23-147G23 (probe on the centromeric side from the MHC region on mouse chromosome 17), Purple: RP23-119G24 (probe on the telomeric side from the MHC region on mouse chromosome 17).

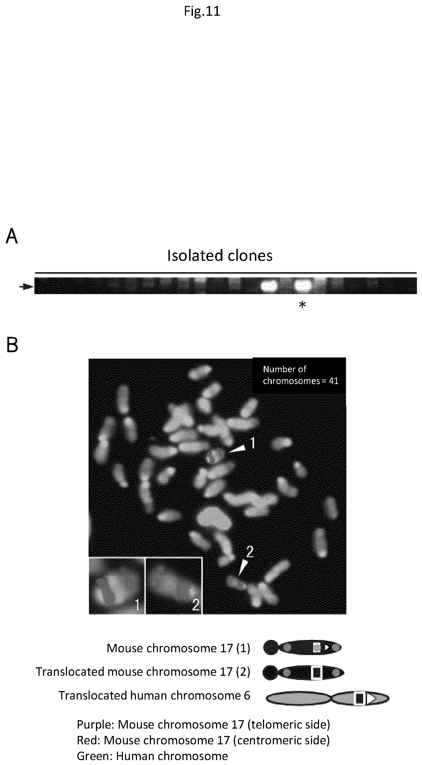

[Figure 11] Views showing the isolation of TV3-4RENKATlb clone. (A) Chromosomal translocation was analyzed by genomic PCR. * represents TV3-4RENKATlb#38 clone. (B) TV3-4RENKATlb#38 was analyzed by FISH. The numbers shown in the FISH images correspond to the numbers assigned to the translocated chromosomes shown in the schematic view. Red: RP23-147G23 (probe on the centromeric side from the MHC region on mouse chromosome 17), Green: human Cot-1 (probe of human genome sequence), Purple: RP23-306H20 (probe on the telomeric side from the MHC region on mouse chromosome 17).

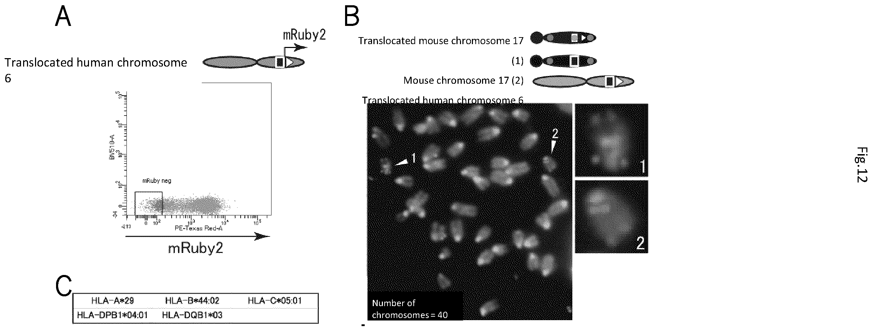

[Figure 12] Views showing the isolation of TV3-4RENKATlbdh6 clone. (A) Sorting of TV3-4RENKATlbdh6. (B) TV3-4RENKATlb#38dh6#3 was analyzed by FISH. The numbers shown in the FISH images correspond to the numbers assigned to the translocated chromosomes shown in the schematic view. The number of chromosomes was confirmed to be 40. Red: RP23-147G23 (probe on the centromeric side from the MHC region on mouse chromosome 17), Green: human Cot-1 (probe of human genome sequence), Purple: RP23-361C19 (probe on the telomeric side from the MHC region on mouse chromosome 17). (C) Results obtained by analyzing the HLA haplotype of TV3-4RENKATlbdh6#3 by whole genome sequencing.

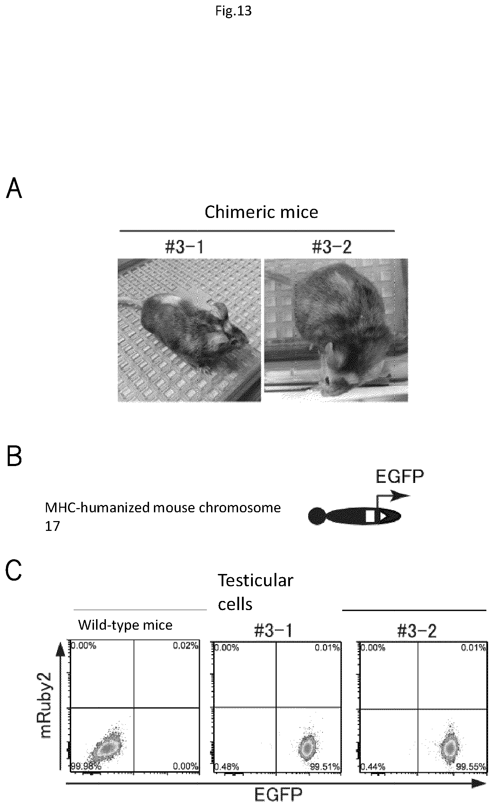

[Figure 13] Views showing MHC-humanized chimeric mice. (A) Appearance of TV3-4RENKATlbdh6#3-derived chimeric mice. (B) Structure of MHC-humanized mouse chromosome 17. An EGFP expression cassette was inserted on the distal side from the MHC region. (C) EGFP expression in testicular cells of TV3-4RENKATlbdh6#3-derived chimeric mice was analyzed by flow cytometry.

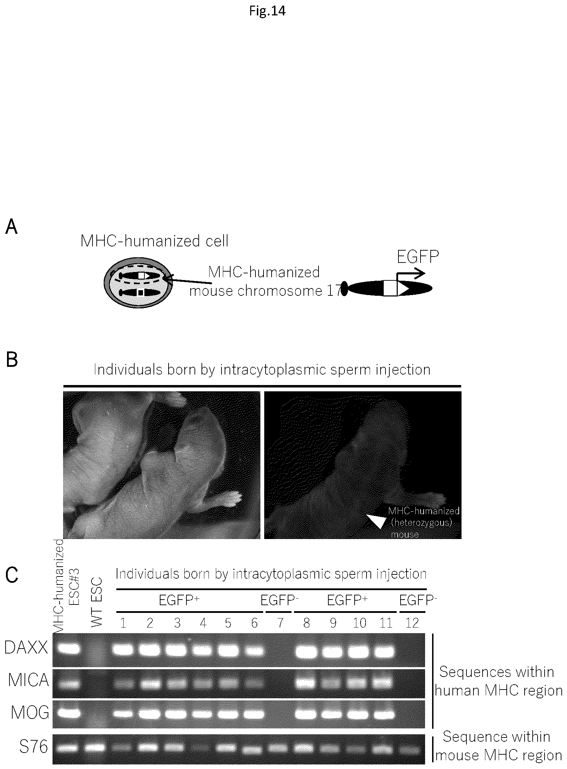

[Figure 14] Views showing generation of MHC-humanized (heterozygous) mice. (A) MHC-humanized (heterozygous) cells had MHC-humanized mouse chromosome 17 with an EGFP expression cassette inserted on the distal side from the MHC region. (B) Individuals born by intracytoplasmic sperm injection using testicular cells from TV3-4RENKATlbdh6#3-derived chimeric mice. Left: Bright field image. Right: EGFP fluorescence was detected. (C) Genomes were prepared from the offsprings obtained by intracytoplasmic sperm injection, and analyzed by PCR for the presence of a sequence in the human MHC region. Individuals 7 and 12 were EGFP-negative offsprings.

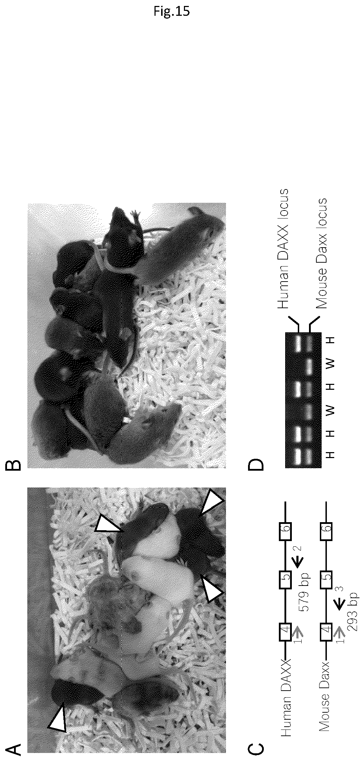

[Figure 15] Views showing generation of MHC-humanized (heterozygous) mice from XO ES cells. (A) Chimeric mice generated from TV3-4XOTlbdh6#11-1-4-57-3 clones. Arrowheads: 100% chimeric mice. (B) Offsprings from XO chimeric mouse (female) and C57BL/6 (male). Black-haired individuals cannot be born unless XO ES cells undergo germline transmission. (C) Genome typing method targeting the DAXX locus within the MHC region. Whether the MHC region was of human or mouse origin was determined using three primers 1-3. Red arrows indicate the locations of primers designed for the part of the sequence that match in human and mouse. Black arrows indicate human- or mouse-specific primers. (D) Examples of genome typing of the offsprings obtained by breeding XO chimeric mouse (female) with C57BL/6 (male). W: Wild-type mice, H: MHC-humanized (heterozygous) mice.

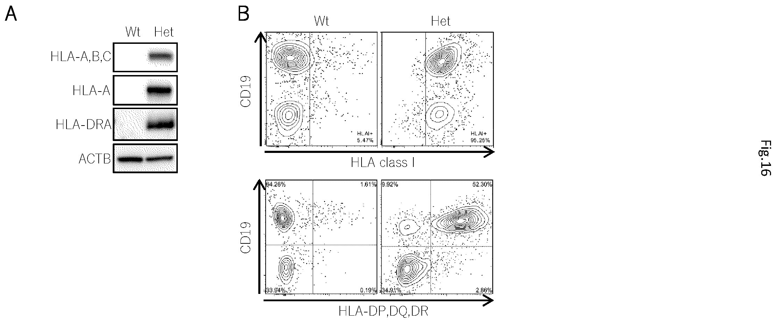

[Figure 16] Views showing the results of expression analysis of human MHC proteins in MHC-humanized (heterozygous) mice. (A) Expression of human MHC proteins in splenocytes was analyzed by Western blotting. Wt, splenocytes from wild-type mouse; Het, splenocytes from MHC-humanized (heterozygous) mouse. (B) Expression of human MHC proteins on the surface of splenocytes was analyzed by flow cytometry. Wt, splenocytes from wild-type mouse; Het, splenocytes from MHC-humanized (heterozygous) mouse.

### [DESCRIPTION OF EMBODIMENTS]

### 1. Overview

The present invention relates to targeting vectors for constructing a non-human chromosome in which the non-human MHC region has been replaced with the human MHC region (MHC-humanized chromosome), to a non-human mammal having said MHC-humanized chromosome, and a method for producing the same.

If a non-human animal with a chromosome incorporating the human MHC region can be generated, genes that define the MHC (MHC genes) can be expressed by an endogenous promoter. This enables reconstruction of a human immune system that simulates the physiological conditions, which is useful for drug discovery and the study of human immune responses.

However, since the length of the human MHC region is nearly 3.4 Mb, it is not possible to introduce the MHC region, especially its entire region, by a transgenic method using a bacterial artificial chromosome (BAC), etc. In addition, the MHC region has a very high gene density, and contains many genes other than the MHC genes.

Therefore, in the present invention, the present inventors attempted to create a non-human animal with a humanized MHC region by replacing the non-human MHC region with the human MHC region using chromosomal engineering.

An overview of the replacement of the non-human mammalian MHC region with the human MHC region is shown in Figure 1.

In Figure 1, (a) is a schematic view of human chromosome 6 and a non-human chromosome before the replacement and (c) is a schematic view of chromosomes after the replacement of the MHC region. The replacement of the MHC region is performed using either a two-step recombination consisting of translocational recombination from (a) to (b1) and translocational recombination from (b1) to (c) (which is referred to as "two-step recombination 1") or a two-step recombination consisting of translocational recombination from (a) to (b2) and translocational recombination from (b2) to (c) (which is referred to as "two-step recombination 2"). In the present invention, the order of recombination is arbitrary, and either two-step recombination 1 or two-step recombination 2 can be used.

In Figure 1, the telomeric side is referred to as the distal side and the centromeric side is referred to as the proximal side with respect to the MHC region, i.e., the target of the translocational recombination (also referred to herein as "replacement" or simply "recombination"). An arbitrary position located on the distal side from the human MHC region, which defines the boundary of recombination located between the human MHC region and the distal end, is designated as P1, and an arbitrary position located on the distal side from the non-human MHC region, which defines the boundary of recombination located between the non-human MHC region and the distal end and corresponds to the aforementioned P1, is designated as p1. Moreover, in Figure 1, an arbitrary position located on the proximal side from the human MHC region targeted for recombination, which defines the boundary of recombination located between the human MHC region and the centromere, is designated as P2, and an arbitrary position located on the proximal side from the non-human MHC region, which is the boundary of recombination located between the non-human MHC region and the centromere and corresponds to the aforementioned P2, is designated as p2.

Accordingly, in the two-step recombination 1, the recombination from (a) to (b1) is the first recombination, which is the recombination between the region extending from P1 to the distal end (telomere) and the region extending from p1 to the distal end (telomere). Then, the recombination from (b1) to (c) is the second recombination, which is the recombination between the region extending from P2 to the distal end (telomere) and the region extending from p2 to the distal end (telomere).

Alternatively, in the two-step recombination 2, the recombination between the region extending from the centromeric side (P2) with respect to the human MHC region, i.e., the target of the recombination in Figure 1, to the distal end and the region extending from the centromeric side (p2) with respect to the non-human MHC region to the distal end is the "first recombination" (Figure 1(b2)). At this stage, recombination between the region extending from P1 to the distal end of the human chromosome and the region extending from p1 to the distal end of the non-human chromosome has not occurred. The subsequent recombination between the region extending from P1 to the distal end of the human chromosome and the region extending from p1 to the distal end of the non-human chromosome is the "second recombination".

After the recombination is complete, MHC-humanized cells, e.g., ES cells, can be created and transplanted into a foster parent to create a chimeric non-human animal. After the recombination is complete, it is preferable to remove the residual human chromosomes in the cells. Human chromosomes can be eliminated and removed by passaging in the absence of selective agents. While the chromosomes of non-human mammals are diploid chromosomes, among the non-human mammalian chromosomes after the recombination is complete according to the present invention, those in which a MHC region has been replaced with a MHC region on human chromosome may be either homozygous (two chromosomes are humanized) or heterozygous (one chromosome is humanized).

Thus, a MHC-humanized heterozygous animal can be created by subsequent natural breeding, intracytoplasmic sperm injection, or the like and a MHC-humanized homozygous animal can be further created by natural breeding, intracytoplasmic sperm injection, or the like. Herein, the term "animal" refers to a non-human mammal unless otherwise specified.

Here, human MHC is the same molecule as HLA and genes that determine this molecule are arranged in series on chromosome 6. The MHC regions of non-human mammals differ from animal to animal, and they are located, for example, on chromosome 17 in mice, on chromosome 20 in rats, on chromosome 12 in rabbits, and on chromosome 7 in pigs.

Note that the term "MHC region" is sometimes used to describe genes that encode MHC in a gene region. Since the mouse MHC region is located on the long arm, for example, when the non-human chromosome in Figure 1 represents a mouse chromosome, the orientation of the long and short arms of the mouse chromosome is opposite to the orientation of the long and short arms of the human chromosome.

The MHC region targeted for recombination may be the entire MHC region or a portion thereof. With respect to class I, it may be either a classical class I molecule or a non-classical class I molecule. In humans, there are three types of classical class I molecules HLA-A, HLA-B, and HLA-C and non-classical class I molecules HLA-E, HLA-F, HLA-G, MICA, and MICB.

Human class II genes are located in the human MHC class II region, and the DR, DQ and DP loci encode the majority of products in the region. Although the mouse MHC also includes mouse H2-M2 and H2-M3, these MHCs do not need to be included in the region targeted for the replacement in the present invention. The class III region contains a variety of genes other than MHC and it is a region located on the distal side from HLA-DRA and on the proximal side from MICB in human.

For example, Figure 2 is a schematic view showing the human MHC region. The MHC region targeted for replacement may be only a class I region, only a class II region, only a class III region, or a combination of these regions. The class I region may be a region containing a classical class I MHC gene, a region containing a non-classical class I MHC gene, or both regions. Thus, according to the present invention, the region forming each class, may be, for example, in the case of class I, any one of HLA-A, HLA-B, HLA-C, HLA-E, HLA-F, HLA-G, MICA, MICB, or a combination of two or more thereof, or, in the case of class II, any one of HLA-DP, HLA-DQ, HLA-DR, HLA-DO, HLA-DM, or a combination of two or more thereof. The class III region contains a variety of genes other than MHC and it is a region located on the distal side from HLA-DRA and on the proximal side from MICB in humans.

Here, the MHC region targeted for recombination may be the entire MHC region or a portion thereof as described above. Therefore, depending on the selection of the targeted MHC region, P1, p1, P2, or p2 may be located in the unselected MHC region.

According to the present invention, in the case of the human MHC class I region, the entire MICB-HLA-F region (region designated as L2 in Figure 2), and, in the case of the class II region, the entire HLA-DP-HLA-DR region (region designated as L1 in Figure 2) is preferably contained. The class III region is a region located on the distal side from HLA-DRA and on the proximal side from MICB (region designated as L3 in Figure 2). In one aspect of the present invention, it is more preferable to replace the entire MHC region shown in Figure 2 (region designated as L4 in Figure 2).

### 2. Targeting vectors

### (1) First recombination in two-step recombination 1 and second recombination in two-step recombination 2

According to the present invention, vectors used to cause recombination are called targeting vectors. The targeting vectors used here are not limited as long as they can cause the above recombination, and they may be vectors that utilize recombinase recognition sequences and recombinases, or vectors that utilize genome editing tools. The genome editing tool can be used as a tool to cleave at the aforementioned positions P1 and p1, as necessary. Since the genome editing tool can be used to cleave a chromosome at a desired position, it can facilitate subsequent recombination.

### <Recombination using recombinase>

For recombination using recombinase, for example, a recombinase recognition sequence called loxP and a recombinase called Cre, are used. The Cre/loxP-specific recombination system is well known. Cre enzyme is a protein consisting of 343 amino acids derived from P1 phage, which recognizes a specific 34 bp nucleotide sequence called the loxP site and is capable of site-specific recombination. The loxP site can be divided into three parts with 13, 8, and 13 nucleotides, and the sequences with 13 nucleotides have complementary inverted repeat sequence structures. Meanwhile, the sequence with 8 nucleotides plays a role in determining the orientation of the loxP site. Many mutant loxPs called Lox511 and lox2272 are also known, and it is also possible to use these recombinase recognition sequences.

In another aspect of the present invention, other examples of site-specific recombination systems using site-specific recombinases, besides the Cre/loxP system, include Flp/FRT system derived from yeast plasmid 2µ, PhiC31 integrase system derived from bacteriophage PhiC31, and Bxb1 integrase system derived from bacteriophage Bxb1.

In Figure 1, the targeting vectors used for performing recombination of (a) to (b1) or (b2) to (c) are a combination of a targeting vector 1 and a targeting vector 2 below, or a targeting vector 3 below. The combination of the targeting vector 1 and the targeting vector 2 refers to vectors that causes recombination using a recombinase recognition sequence and a recombinase, and the targeting vector 3 refers to a vector that causes recombination using a genome editing tool.

### (i) Targeting vector 1

The targeting vector 1 is incorporated into human chromosome 6 at an arbitrary position P1 located on the distal side from the MHC (human MHC) region targeted for recombination on the short arm of the human chromosome 6. The targeting vector 1 causes translocational recombination between a region that extends from the aforementioned P1 to the distal end and a region in non-human mammalian chromosome, the region extending from position p1, which corresponds to the aforementioned P1 and is located on the distal side from the non-human MHC region targeted for recombination, to the distal end. Moreover, the targeting vector 1 contains a gene cassette 1 containing a recombinase recognition sequence, one partial sequence of a drug resistance gene 1 for drug selection, which is reconstituted upon the translocational recombination, and a drug resistance gene 2 for drug selection after targeting;

The gene cassette 1 comprises a construct in which, for example, the recombinase recognition sequence is loxP, the drug resistance gene 1 is neomycin-resistance gene, and the drug resistance gene 2 is hygromycin-resistance gene.

Here, "corresponding" is a term that refers to the positional relationship of the locations where the recombination takes place. Specifically, the aforementioned P1, p1, P2, and p2 are markers that indicate the positional relationship, i.e., whether the site is on the distal or proximal side as seen from the MHC region targeted for recombination. Therefore, it does not require that the genes adjacent to P1 and p1 or P2 and p2 are identical. In some mammalian species, the MHC region may be inverted with respect to the orientation of the human MHC region. In this case, when loci on both sides of the site (P1) where the targeting vector is to be incorporated are the MOG locus and the GABBR1 locus for the human loci (see example and Figure 5), p1 of an animal with an inverted MHC region is located, for example, between the Kifc1 locus and the Daxx locus.

Figure 5 is a schematic view showing structures of the targeting vectors used in an example of the present invention. For ease of explanation, targeting vectors in the case where a mouse MHC region is replaced with a human MHC region will be described as an example.

In Figure 5A, "TV3R" represents the aforementioned gene cassette 1. The recombinase recognition sequence, which is loxP sequence or the like, is indicated by the black sideways triangle in the figure. One partial sequence of the drug resistance gene 1 for drug selection, which is reconstituted upon translocational recombination (a predetermined partial sequence of the drug resistance gene 1 used in the targeting vector 1) is "3'neo" according to the present invention. In this case, the drug resistance gene is neomycin-resistance gene. The drug resistance gene 2 for drug selection after targeting is designated as "Hyg", and the drug resistance gene in this case is hygromycin-resistance gene.

The gene cassette 1 generated in the example of the present invention contains a sequence represented by the following formula (1).

BGHpA-3'Neo-SA-loxP-EF1-Hyg-P2A-mRuby2-RGpA (1)

In formula (1), BGHpA represents bovine growth hormone-derived poly A addition signal sequence, 3'Neo represents a part of the 3' sequence of neomycin-resistance gene, SA represents a splicing acceptor sequence, loxP represents a recombinase recognition sequence, EF1 represents human elongation factor 1α-derived promoter sequence, Hyg represents hygromycin-resistance gene, P2A represents porcine teschovirus-derived 2A sequence, mRuby2 represents red fluorescent gene, and RGpA represents rabbit β-globin-derived poly A addition signal sequence. Note that, according to the present invention, Flp recombinase recognition sequence (FRT) may be added to the cassette 1 represented by (1) above.

"TV3R-dHLA" is the targeting vector 1, in which sequences homologous to the sequences of the regions on both sides of the position (P1), where TV3R is to be incorporated, are linked to both sides of TV3R.

In Figure 5A, "PX458a-dHLACR1" is a genome editing vector that artificially cleaves the chromosome at an arbitrary position P1 to facilitate recombination with the targeting vector 1. Any known tool can be used as the genome editing tool used for the genome editing vector. Examples include tools using zinc finger nuclease (ZFN), TALE nuclease (TALEN), and CRISPR-Cas9 (CRISPR-Cas9).

PX458a-dHLACR1 is a CRISPR-Cas9-based vector that is composed of, for example, a guide RNA sequence, a Cas9 sequence, and a gene sequence for fluorescent protein ametryn.

Figure 1(d) shows an enlarged view of the region extending from near the MHC region to the distal end. In the example of the present invention, the targeting vector 1 was designed to be incorporated between the MOG locus and the GABBR1 locus on the human chromosome (Figure 5A).

### (ii) Targeting vector 2

The targeting vector 2 is incorporated into non-human mammalian chromosome in which the MHC region exists, at position p1 that corresponds to the aforementioned P1 and that is located on the distal side from the non-human MHC region targeted for recombination.

In the mouse used in the example, the targeting vector 2 causes translocational recombination between a region that extends from the aforementioned p1 to the distal end and the region that extends from the aforementioned P1 to the distal end on the human chromosome. Moreover, it contains a gene cassette 2 containing a recombinase recognition sequence, the other partial sequence of the drug resistance gene 1 for drug selection, which is reconstituted upon the translocational recombination (a partial sequence other than the predetermined partial sequence of drug resistance gene 1 used in the targeting vector 1), and a drug resistance gene 3 for drug selection after targeting.

The gene cassette 2 comprises a construct in which, for example, the recombinase recognition sequence is loxP, the drug resistance gene 1 is neomycin-resistance gene, and the drug resistance gene 3 is puromycin-resistance gene.

As mentioned above, in some animal species, the MHC region may be inverted. In this case, the targeting vector 2 can still be incorporated into position p1 that corresponds to the arbitrary position P1.

In Figure 5B, "TV4" represents the gene cassette 2. The recombinase recognition sequence, which is loxP sequence or the like, is indicated by the black sideways triangle in the figure. The partial sequence of drug resistance gene 1 for drug selection, which is reconstituted upon the translocational recombination is "5'neo" according to the present invention. In this case, the drug resistance gene is neomycin-resistance gene. The drug resistance gene 2 for drug selection after targeting is designated as "Puro", and the drug resistance gene in this case is puromycin-resistance gene.

The gene cassette 2 generated in the example of the present invention contains a sequence represented by the following formula (2).

EF1-EGFP-P2A-5'Neo-SD-loxP-SA-T2A-Puro-RGpA (2)

In Formula (2), EF1 represents human elongation factor 1α-derived promoter sequence, EGFP represents green fluorescent gene, P2A represents porcine teschovirus-derived 2A sequence, 5'Neo represents a part of the 5' sequence of neomycin-resistance gene, SD represents a splicing donor sequence, loxP represents a recombinase recognition sequence, SA represents a splicing acceptor sequence, T2A represents Thosea asigna virus-derived 2A sequence, Puro represents puromycin-resistance gene, and RGpA represents rabbit β-globin-derived poly A addition signal sequence. Note that, according to the present invention, FRT may be added to the cassette 2 represented by Formula (2) above.

TV4-dH2 is the targeting vector 2, in which sequences homologous to the sequences of the regions on both sides of the position (p1), where TV4 is to be incorporated, are linked to both sides of TV4.

In Figure 5B, "PX458a-dH2CR1" is a genome editing vector that artificially cleaves the chromosome at an arbitrary position p1 to facilitate recombination with the targeting vector 2. The genome editing tool used for the genome editing vector is the same as described above. PX458a-dH2CR1 is a CRISPR-Cas9-based vector that is composed of, for example, a guide RNA sequence, a Cas9 sequence, and a gene sequence for the fluorescent protein ametryn.

As in Figure 1(d), in the example of the present invention, the targeting vector 2 was designed to be incorporated between the Mog locus and the Gabbr1 locus on the non-human chromosome (Figure 5B).

### <Recombination using genome editing vector>

### (iii) Targeting vector 3

In another aspect of the present invention, a genome editing tool can be used instead of recombination using recombinases to perform the first recombination. The targeting vector 3 that causes translocational recombination between a region on human chromosome 6, the region extending from an arbitrary position P1, which is located on the distal side from a human MHC region targeted for recombination and targeted for cleavage by a genome editing tool, to the distal end, and a region on a non-human mammalian chromosome in which MHC exists, the region extending from position p1, which corresponds to the aforementioned P1 and is located on the distal side from the non-human MHC region, to the distal end.

Again, replacement of the MHC region can be performed using either the translocational recombination from (a) to (b1) and from (b1) to (c) (two-step recombination 1) or the translocational recombination from (a) to (b2) and from (b2) to (c) (two-step recombination 2) as shown in Figure 1.

The targeting vector 3 contains a homologous sequence of the sequence of a partial region (this region is called "R1a") located between the aforementioned P1 and the human MHC region, a drug resistance gene 4 for drug selection after targeting, and a homologous sequence of the sequence of a partial region (this region is called "R2b") located on the distal side from the aforementioned p1, or the targeting vector 3 contains a homologous sequence of the sequence of a partial region (this region is called "R1b") located on the distal side from the aforementioned P1, a drug resistance gene 4 for drug selection after targeting, and a homologous sequence of the sequence of a partial region (this region is called "R2a") located between the aforementioned p1 and the non-human MHC region.

The positional relationships of R1a, R1b, R2a and R2b are shown in Figure 3(a). Regions R1a, R1b, R2a and R2b can be arbitrarily positioned with respect to P1 and p1, but it is preferred that they are near P1 and p1.

The targeting vector 3 used in the present invention can comprise, for example, the R2a sequence, a CAG promoter sequence, blasticidin-resistance gene, rabbit β-globin-derived poly A addition signal sequence, and the R1b sequence.

(2) Second recombination in two-step recombination 1 or first recombination in two-step recombination 2

According to the present invention, a vector that causes the second recombination in the two-step recombination 1 and the first recombination in the two-step recombination 2, i.e., recombination from (b1) to (c) in Figure 1 or recombination from (a) to (b2) in Figure 1, is called a targeting vector 4.

Although recombination in this section can be performed using targeting vectors utilizing recombinases as described above, preliminary experiments by the present inventors have shown that recombination occurs more efficiently and the subsequent creation of non-human mammals is also more effective using a genome editing tool-based targeting vector than using recombinase-based targeting vectors.

Therefore, according to the present invention, the second recombination in the two-step recombination 1 or the first recombination in the two-step recombination 2 is performed by using a genome editing tool.

### (iv) Targeting vector 4

In the case of the two-step recombination 1, in the chromosome after the translocational recombination by the actions of the targeting vector 1 and the targeting vector 2 or by the action of the targeting vector 3 (Figure 1(b1)), the targeting vector 4 causes translocational recombination between a region extending from an arbitrary position P2, which is targeted for cleavage by a genome editing tool and located on the proximal side from a human MHC region targeted for recombination, to the distal end and a region on the non-human mammalian chromosome in which the non-human MHC exists, the region extending from position p2, which corresponds to the aforementioned P2 and is located on the proximal side from the non-human MHC region, to the distal end.

Alternatively, in the case of the two-step recombination 2, the targeting vector 4 can be used first, followed by the targeting vectors 1-3. In short, the recombination shown in Figure 1(a)-(c) can be performed by first recombining between regions extending from P1 or p1 to the distal ends, then recombining between regions extending from P2 or p2 to the distal ends (in the order of (a), (b1), and (c) in Figure 1), or by first recombining between regions extending from P2 or p2 to the distal ends, and then recombining between regions extending from P1 or p1 to the distal end (in the order of (a), (b2), and (c) in Figure 1).

The targeting vector 4 contains a homologous sequence of the sequence of a partial region R3a located on the proximal side from the aforementioned P2 (between P2 and the centromere), a drug resistance gene 5 for drug selection after targeting (e.g., blasticidin-resistance gene), and a homologous sequence of the sequence of a partial region R4b located between the aforementioned p2 and the non-human MHC region, or the targeting vector 4 contains a homologous sequence of the sequence of a partial region R3b located between the aforementioned p2 and the human MHC region, a drug resistance gene 5 for drug selection after targeting, and a homologous sequence of the sequence of a partial region R4a located on the proximal side from the aforementioned p2 (between p2 and the centromere).

The positional relationships of R3a, R3b, R4a and R4b are shown in Figure 3(b). Regions R3a, R3b, R4a and R4b can be arbitrarily positioned with respect to P2 and p2, but it is preferred that they are near P2 and p2.

The targeting vector 4 used in the present invention can comprise, for example, the R3a sequence, a CAG promoter sequence, blasticidin-resistance gene, rabbit β-globin-derived poly A addition signal sequence, and the R4b sequence.

Figure 1(e) shows an enlarged view of the region extending from near the MHC region to the distal end. In the example of the present invention, the targeting vector 4 was designed such that recombination occurs between the KIFC1 locus and the DAXX locus on the human chromosome and between the Kifc1 locus and the Daxx gene locus on the non-human chromosome (Figure 5C). The targeting vector 4 contains blasticidin (BS)-resistance gene.

In Figure 5C, "PX458.1a-pHLACR2" is a genome editing vector that artificially cleaves the chromosome at an arbitrary position P2 to facilitate translocational recombination mediated by the targeting vector 4. PX458.1a-pHLACR2 is a CRISPR-Cas9-based vector that is composed of, for example, a guide RNA sequence, a Cas9 sequence, and a gene sequence for fluorescent protein ametryn.

Similar to PX458.1a-pHLACR2, "PX458.1a-pH2CR1" is a genome editing vector that artificially cleaves the chromosome at an arbitrary position p2 to facilitate translocational recombination mediated by the targeting vector 4. PX458.1a-pH2CR1 is a CRISPR-Cas9-based vector that is composed of, for example, a guide RNA sequence, a Cas9 sequence, and a gene sequence for fluorescent protein ametryn.

### <Drug resistance genes for drug selection>

According to the present invention, drug resistance genes 1-5 for drug selection are used in the targeting vectors as described above. The combination of the drug resistance genes 1-5 is arbitrary, and is selected appropriately to achieve the drug selection of interest.

Examples of the genes used as the drug resistance genes 1-5 include, but are not limited to, neomycin-resistance gene, hygromycin-resistance gene, puromycin-resistance gene, and blasticidin-resistance gene.

### <Fluorescent genes for confirming recombination or selection>

According to the present invention, a predetermined fluorescent gene can be contained in the targeting vector to confirm chromosome transfer, to confirm that the transferred chromosome is maintained, or to confirm that the drug selection was performed as intended. The type of the fluorescent gene can be selected as desired as long as the confirmation can be done in each process, and it is possible to use different types of fluorescent genes for the respective vectors.

Examples of the fluorescent genes include green fluorescent genes (GFP, EGFP, etc.), red fluorescent genes, and yellow fluorescent genes.

### 3. Cells

To establish a non-human mammal with humanized MHC, it is necessary to replace an endogenous MHC gene with a human MHC gene in non-human mammalian cells.

According to the present invention, ordinary homologous recombination or genome editing techniques can be used as described above.

The cells are not particularly limited as long as they are animal cells, and examples include ES cells, sperm stem cells, and fibroblasts, preferably ES cells.

Examples of the cell culture medium include GMEM medium (Glasgow's Minimal Essential Medium), DMEM (Dulbecco's Modified Eagle Medium), and RPMI 1640 medium. Generally used animal cell culture additives such as fetal bovine serum (FBS), nonessential amino acids, antibiotics (e.g., penicillin, streptomycin, etc.), and growth factors/cytokines (epidermal growth factor, fibroblast growth factor, leukemia inhibitory factor, etc.) can be added as appropriate to the culture medium according to the cell type.

After culturing the cells for a predetermined period of time, the cells are recovered by incubating them in a medium containing trypsin. The recovered cells may be passaged multiple times in the presence or absence of feeder cells, as needed. To confirm that the cultured cells are the cells of interest, their marker genes can be used as indicators. If the cells are ES cells, for example, Oct3/4, alkaline phosphatase, Nanog, etc. can be used as an indicator and detected by any method such as RT-PCR or Western blotting. ES cells can also be judged by their colony morphology.

### 5. Preparation of non-human mammal

Non-human mammals can be generated by a standard method such as somatic cell nuclear transfer, intracytoplasmic sperm injection using sperm stem cells, etc., and also by generating chimeric animals using ES cells.

The non-human mammals to be generated are not particularly limited and examples include rodents, domestic animals, and primates. Examples of the rodents include mice, rats, guinea pigs, and hamsters; examples of the domestic animals include bovines, horses, pigs, and sheep; and examples of the primates include Japanese macaques and common marmosets. In addition, pet or experimental animals such as dogs, cats, monkeys, and rabbits can also be used.

According to the present invention, rodents, especially mice, are preferred.

In the case of a chimeric animal, the aforementioned established ES cells are first aggregated with a 8-cell embryo or injected into a blastocyst. The embryo generated in this way is called a chimeric embryo, and this chimeric embryo is transferred into the uterus of a pseudopregnant foster parent to give birth to a chimeric animal.

Here, an "embryo" refers to an individual at stages from fertilization to birth in ontogeny, and includes a 2-cell stage embryo, a 4-cell stage embryo, a 8-cell stage embryo, a mulberry embryo, a blastocyst, etc.

Hereinafter, ES cells will be described as an example for convenience of explanation.

As a method for generating an aggregate using ES cells and an embryo, a known method such as the microinjection method or the aggregation method can be used.

If the microinjection method is employed, ES cells are injected into the recovered embryo to create a cell aggregate. If the aggregation method is employed, ES cells can be aggregated by sprinkling them over a normal embryo from which the zona pellucida has been removed. The ES cells used here are not particularly limited, and examples include C57BL/6-derived RENKA, Y chromosome-deficient strain XO of TT2 cells derived from an F1 hybrid of C57BL/6 and CBA, and ES cells established from an F1 hybrid of C57BL/6 and DBA/sCrSlc.

Meanwhile, a pseudopregnant female animal for use as a foster parent can be obtained by breeding a female animal with normal menstrual cycle with a male animal that has been castrated by vasoligation or other means. The chimeric embryo generated by the method described above is transferred into the uterus of the created pseudopregnant animal, and then the animal is allowed to give birth to chimeric animals.

Among such chimeric animals, animals derived from the ES cell-transplanted embryo are selected. The selected chimeric animals are crossed with animals of inbred strains. The appearance of the coat color of the animal derived from the ES cells in the born offsprings confirms that the ES cells have been introduced into the germ line of the chimeric animals.

Whether or not the born offsprings have the humanized MHC gene can be identified by cleaving the DNA by a restriction enzyme to see if a DNA fragment of the desired size is detected, or by PCR method.

### EXAMPLES

Hereinafter, the present invention will be described further in detail by way of examples. The scope of the present invention, however, should not be limited to these examples.

### 1. Generation of targeting vectors

### <Methods>

### Methods

### Generation of TV3R-dHLA

pEF-GFP (Addgene plasmid #11154; http://n2t.net/addgene:11154; RRID: Addgene_11154) was digested with restriction enzymes EcoRI-HF (New England BioLabs) and NotI-HF (New England BioLabs), and hygromycin-resistance gene was cloned downstream of human elongation factor 1α-derived promoter sequence using In-Fusion (registered trademark) HD Cloning Kit (Clontech) according to the attached protocol to generate pEF-Hyg (SEQ ID NO:1). Next, the upstream side of the human elongation factor 1α-derived promoter sequence encoded by pEF-Hyg was digested with restriction enzyme SalI, and synthetic DNA fragment FRT-BGHpA-3'Neo-SA-loxP (Thermo Fisher Scientific) (SEQ ID NO: 2) was cloned using In-Fusion (registered trademark) HD Cloning Kit to produce TV3.

Then, a sequence including the 5' and 3' homologous regions of the targeting vector TV3R-dHLA was amplified using the following primer set with the genome of MRC5 cell as a template using PrimeSTAR (registered trademark) GXL DNA Polymerase (Takara) according to the attached protocol.
hMOG CR LA fw1: AATTAGCCATGTGTGGTGGCACACG (SEQ ID NO:3)
hMOG CR RA rv1: CCCTGAGAGTCCTGTGCATATCAGC (SEQ ID NO:4)

Furthermore, the entire homologous region was amplified by Nested-PCR method using the following primer set and cloned into pGEM (registered trademark)-T Easy Vector (Promega) to generate pGEMTe-dHLA.
hMOG nest fw1: TGAGGCAGGAGAATCACTTG (SEQ ID NO:5)
hMOG nest rv1: GCTAAGGATTAAATCACTGCGGTTATC (SEQ ID NO:6)

Next, pGEMTe-dHLA was amplified using the following primer set and PrimeSTAR (registered trademark) GXL DNA Polymerase, and a fragment containing FRT-BGHpA-3'Neo-SA-loxP prepared by digesting TV3 with SalI-HF (New England BioLabs) and HindIII-HF (New England BioLabs) was cloned using In-Fusion (registered trademark) HD Cloning Kit to generate TV3-dHLA.

TV3-dHLA inf fw1: GACCTGCAGCCCAAGCTACCAGGGATAACAGGGGAACAG (SEQ ID NO:7)

TV3-dHLA inf rv1: GAATAGGAACTTCGTCGACTGTCAAGCAGCTAGCAGGC (SEQ ID NO:8)

Synthetic DNA fragment P2A-mRuby2 (SEQ ID NO:9) (Thermo Fisher Scientific) was further amplified using the following primer set and PrimeSTAR (registered trademark) GXL DNA Polymerase. The amplified product and TV3R-dHLA were digested with restriction enzyme BssHII (New England BioLabs) and both were ligated using Ligation-Convenience Kit (Nippon gene) to generate TV3R-dHLA.
RGpA seq fw1: ACTACTCCCAGTCATAGCTG (SEQ ID NO:10)
BGHpA seq rv1: CTATTGTCTTCCCAATCCTCC (SEQ ID NO:11)

### Generation of TV4-dH2

pEF-GFP (Addgene plasmid #11154; http://n2t.net/addgene:11154; RRID: Addgene_11154) was digested with restriction enzymes EcoRI-HF and NotI-HF, and EGFP gene was cloned downstream of human elongation factor 1α-derived promoter sequence using In-Fusion (registered trademark) HD Cloning Kit according to the attached protocol to generate pEF-EGFP2 (SEQ ID NO:12). Next, pEF-EGFP2 was digested with restriction enzyme BsrGI (New England BioLabs), into which sequence P2A-5'Neo-SD-loxP-SA-T2A-Puro (Thermo Fisher Scientific) (SEQ ID NO: 13), which was synthesized as two DNA fragments, was cloned using In-Fusion (registered trademark) HD Cloning Kit to generate TV4dF.

TV4dF was digested with restriction enzyme SalI-HF, into which the following annealed oligo DNA was ligated using Ligation-Convenience Kit to generate TV4.

FRT fw:
TCGAGGAAGTTCCTATTCCGAAGTTCCTATTCTCTAGAAAGTATAGGAACTT
CG (SEQ ID NO:14)

FRT rv:
TCGACGAAGTTCCTATACTTTCTAGAGAATAGGAACTTCGGAATAGGAACTT
CC (SEQ ID NO:15)

Then, a sequence including the 5' and 3' homologous regions of the targeting vector TV4-dH2 was amplified using the following primer set with the genome of C57BL/6 mouse as a template using PrimeSTAR (registered trademark) GXL DNA Polymerase according to the attached protocol.
Mog CR LA fw1: GCGTCAGATCTCATTATGGATGGCTG (SEQ ID NO:16)
Mog CRRA rv1: CGAGCCTATGAGGGTCATTCTCACTC (SEQ ID NO: 17)

Furthermore, the entire homologous region was amplified by Nested-PCR method using the following primer set and cloned into pGEM (registered trademark)-T Easy Vector (Promega) to generate pGEMTe-dH2.
Mog nest fw1: GAACTCAGGACCTTCAGAAG (SEQ ID NO:18)
Mog nest rv1: CAACTAACACAGTTACTCCCATAAC (SEQ ID NO:19)

Next, pGEMTe-dH2 was amplified using the following primer set and PrimeSTAR (registered trademark) GXL DNA Polymerase, and a fragment containing P2A-5'Neo-SD-loxP-SA-T2A-Puro prepared by digesting TV4 with SalI-HF and HindIII-HF was cloned using In-Fusion (registered trademark) HD Cloning Kit to generate TV4-dH2.
TV4-dH2 inf fw1: GTATAGGAACTTCGTCGAACGCTGGTAACATTTGCCAACATCT (SEQ ID NO:20)
TV4-dH2 inf rv1: ACCTGCAGCCCAAGCTTTGTTGGCCTGATATCTCATTAAATCTG (SEQ ID NO:21)

### Generation of TV-TI1/2-BS

Hprt expression vector TV-Tl1/2-Hprt (SEQ ID NO:22), which has pGEM (registered trademark)-T Easy Vector as the vector backbone and the 5' and 3' homologous regions used for TV-Tll/2-BS, was digested with restriction enzymes XhoI (New England BioLabs) and XbaI (New England BioLabs), into which a DNA fragment (IDT) (SEQ ID NO:23) containing a synthetic blasticidin-resistance gene was cloned using In-Fusion (registered trademark) HD Cloning Kit to generate TV-Tl1/2-BS.

### Generation of genome editing vector (1)

The gRNAs of PX458a-dHLACR1 and PX458a-dH2CR1 were designed for the following sequences, and PX458a, which was generated by modifying pSpCas9(BB)-2A-GFP (PX458) (Addgene plasmid #48138; http://n2t.net/addgene:48138; RRID: Addgene 48138) and replacing EGFP gene with fluorescent gene ametryn (SEQ ID NO:24), was used for expression of gRNA and Cas9.
PX458a-dHLACR1: CTAGCTGCTTGACAGTAACC (SEQ ID NO:25)
PX458a-dH2CR1: GATATCAGGCCAACAAACGC (SEQ ID NO:26)

### Generation of genome editing vector (2)

The gRNAs of PX458.1a-pHLACR2 and PX458.1a-pH2CR1 were designed for the following sequences, and PX458.1a, which was generated by modifying the gRNA scaffold sequence of PX458a, was used for expression of gRNA and Cas9 (SEQ ID NO:27).
PX458.1a-pHLACR2: GACTCATAAACCGGAGGAGC (SEQ ID NO:28)
PX458.1a-pH2CR1: CGTGTAGTAGGCGCCCGTTA (SEQ ID NO:29)

### Preparation of drug-resistant mouse embryonic fibroblasts (MEFs)

Drug-resistant MEFs were generated in order to culture ES cells on feeder cells during drug selection as well. First, MEFs were prepared from a 14.5-day-old embryo and infected with a lentivirus expressing a gene (HBPN) in which hygromycin-resistance gene, blasticidin-resistance gene, puromycin-resistance gene, and neomycin-resistance gene were linked by 2A sequences. To improve the proliferation potential of the MEFs, HBPN-expressing MEFs were cultured under hypoxic conditions of 5% oxygen/5% CO₂, and they were treated with mitomycin C to be used as drug-resistant MEFs.

### Cloning of MRC5-A9 fused cell hChA9

MRC5 cells and A9 cells transfected with Neo-resistance genes using lentivirus were fused using GenomONE-CF (Ishihara Sangyo Kaisha, Ltd.), and Ouabain-resistant/G418-resistant cells were isolated as fused cells. Human cells were Ouabain-sensitive while mouse cells were Ouabain-insensitive, and the fused cells can be selected by adding G418 and Ouabain to the medium. The fused cells with human chromosome 6 were identified by genomic PCR using the following primer set targeting the region between the KIFC1 and DAXX genes.
pHLA probe fw2: AGTAAATCCTGACTGAGCACCTCCTG (SEQ ID NO:30)
pHLA probe rv1: GCTTGCGTGAGGCTGAAGTGTC (SEQ ID NO:31)

In addition, for the detection of human chromosome 6 by FISH, a DNA fragment artificially synthesized by collecting sequences specific for the human chromosome 6 centromere according to the report of Jabs et al. (Am. J. Hum. Genet. 41:374-390, 1987) was used for the preparation of a probe.

The synthesized sequence was as follows.

AAGGAGTTTCTGAGATTGCTTCTGTCTAGCTTTTATGGAAAGATATTTCCTTT
TCTACCATAGGCCTCAAAGCGCTCTTAGTATACACTTCCAAATTCTACAAAG
AGAGTGTTACTAAACCGCTCTCTCAAAGGAAATGTTAAACTCTATGAGTTGA
AACACAGCACACAAACAGTTCTGAGACACTTCTGTCTGCCTTTTATGTGAAG
ACATCCCTTTCCAAAGAATGCCTCCAAGGGTTCAAAATATCCACTTGTAGAC
TTTACAAAGAGAGTGTTCAAAACTTTCTCTACCAAAAGAAAGGTTAAAGAC
GGTGAGTTCAAACGCACACATCACAAAGTTGTTTCTAAGAATCATCTACTAT
GTTCTAGAGATGTTCCTTTCTATCATAGCTCAATGTCTAAATATCCACTGAA
TC (SEQ ID NO:32)

### Cloning of TV3RhChA9

Clones of the MRC5-A9 fused cell targeted with TV3R-dHLA (TV3RhChA9) were generated by transfecting PX458a-dHLACR1 and targeting vector TV3R-dHLA into hChA9 using PEI max (Polysciences). PX458a-dHLACR1 was co-transfected to increase the homologous recombination efficiency of the targeting vector. On the day after the transfection, the vector-transfected cells were sorted using the expression of the fluorescent gene mRuby2 on TV3R-dHLA as an indicator, and mRuby2-positive hygromycin-resistant clones were isolated. Genomic DNA was prepared from the isolated clones, and the recombinantly transfected clones were identified by PCR. Primers used for PCR were as follows. The same analysis was also performed when recloning was performed from the isolated clones.
dHLA 5 typing fw1: CTTGGGGTCCAGAGAAGAAAATCACTC (SEQ ID NO:33)
BGHpA typing fw1: CTCTATGGCTTCTGAGGCGGAAAGAAC (SEQ ID NO:34)

### Cloning of TV4RENKA

TV4RENKA was an ES cell clone generated by transfecting PX458a-dH2CR1 and targeting vector TV4-dH2 into RENKA using Lipofectamine 3000 (Thermo Fisher Scientific). PX458a-dH2CR1 was co-transfected to increase the homologous recombination efficiency of the targeting vector. EGFP-positive clones were isolated, and the recombinantly transfected clones were identified by genomic PCR. XO and BDF1 clones can also be isolated in the same manner.

Primers used for PCR were as follows.
Mog 3'typing fw1: CGGAGCCTCACGCGATGATCTA (SEQ ID NO:35)
Mog 3'typing fw2: AGTACACTGTAGTTGTCCTCAGATACTCC (SEQ ID NO:36)
Mog 3'typing rv1: TCCACCCCAAACATTTCCACTAACTG (SEQ ID NO:37)

### Cloning of TV3-4RENKA

TV3-4RENKA was an ES cell clone generated by introducing modified human chromosome 6 from TV3RhChA9 to TV4RENKA using retro-MMCT method (Suzuki, T.et al. (2016) PloS One, 11(6): e0157187). Cells that became mRuby2-positive and hygromycin-resistant were cloned, and clones with 41 chromosomes were isolated. In FISH analysis, detection was performed using Cy3-labeled mouse Cot-1 and Cy5-labeled RP11-54H13.

### Cloning of TV3-4RENKATl3/4

Cre expression vector was introduced into TV3-4RENKA using Lipofectamine 3000 to induce chromosomal translocation between loxPs. Clones that had underwent the chromosomal translocation were isolated using G418 resistance as an indicator and designated as "TV3-4RENKATl3/4". After genomic PCR confirmed that the recombination had occurred, FISH analysis confirmed that the translocation had occurred between mouse chromosome 17 and human chromosome 6.

Primers used for PCR were as follows.
5Neo_O fw1: TGCCAGGCCAGGATCTGCTG (SEQ ID NO:38)
Neo_O rv1: ATCCGGTGCTTGGCTTGATG (SEQ ID NO:39)

In the FISH analysis, detection was performed using Cy3-labeled RP11-54H13, DY490-labeled RP23-147G23, and Cy5-labeled RP23-119G24, or using Cy3-labeled human Cot-1 and DY490-labeled mouse Cot-1.

### Cloning of TV3-4RENKATlb

For cloning of TV3-4RENKATlb, TV-Tl1/2-BS, PX458.1a-pHLACR2, and PX458.1a-pH2CR1 were co-transfected into TV3-4RENKATl3/4 using Lipofectamine 3000. On the day after the transfection, ametryn-positive cells were sorted, and clones that became resistant to blasticidin were isolated. After genomic PCR confirmed that the recombination had occurred, FISH analysis confirmed that the replacement of the MHC region had occurred.

Primers used for PCR were as follows.
pH2 seq fw: CACATGTCCCTTCTGCCATAAG (SEQ ID NO:40)
Tlb typing rv1: AGAGATCACTGAAGCTGCTAC (SEQ ID NO:41)

In the FISH analysis, detection was performed using Cy3-labeled RP11-147G23, DY490-labeled human Cot-1, and Cy5-labeled RP23-306H20.

### Cloning of TV3-4RENKATlbdh6

In order to isolate TV3-4RENKATlbdh6, in which the residual human chromosome 6 had been eliminated from TV3-4RENKATlb, mRuby2-negative cells were sorted and seeded on feeder cells for clonal isolation. Furthermore, FISH analysis of clones that were found to have 40 chromosomes (39 chromosomes in the case of XO) by karyotyping confirmed that the residual human chromosome 6 had been eliminated. In the FISH analysis, detection was performed using Cy3-labeled RP11-147G23, DY490-labeled human Cot-1, and Cy5-labeled RP23-361C19. TV3-4XOTlbdh6 and TV3-4BDF1Tlbdh6 were also isolated using the same procedure as for TV3-4RENKATlbdh6.

### 2. Generation of MHC-humanized mice

MHC-humanized chimeric mice were generated by the aggregation chimera method using the MHC-humanized ES cells and ICR-derived embryos. To generate chimeric mice using the MHC-humanized ES cells derived from RENKA, MHC-humanized mice were established by isolating sperm cells from the testes and performing intracytoplasmic sperm injection into oocytes of B6/DBA F1 mice or ICR mice. To generate female chimeric mice using the MHC-humanized ES cells derived from XO ES cells, MHC-humanized chimeric mice (MHC-humanized heterozygous mice) were established by breeding with male C57BL/6 mice. The establishment of the MHC-humanized mice was confirmed by EGFP fluorescence and genomic PCR of the offsprings.

Primer sets used for genomic PCR were as follows.
DAXX:
   Fw, TCTAGTCCCTTCAAGGGCTGAG (SEQ ID NO:42)
   Rv, ACAATGTCTCTCTGAAGGCTGTAC (SEQ ID NO:43)
MICA:
   Fw, AACATCACCGTGACATGCAG (SEQ ID NO:44)
   Rv, TGTCTGCCAATGACTCTGAAG (SEQ ID NO:45)
MOG:
   Fw, CAGCTGCAGCAATTACCGGAGTG (SEQ ID NO:46)
   Rv, GAAGGGAGGCATGTCAGTAGGTC (SEQ ID NO:47)
S76:
   Fw, CCTTTGTGACAGCGCACGTT (SEQ ID NO:48)
   Rv, CCAGACACTAGGGCTTTCGT (SEQ ID NO:49)
DAXX (confirmed with 3 primers):
   Human/mouse common primer, CGGCTGGATGAGGTCATCTCCAA (SEQ ID NO:50)
   Mouse-specific primer, GACTCAACTCTGGGAGCTCATG (SEQ ID NO:51)
   Human-specific primer, GTTTCAAACAGGTGGCTCATGC (SEQ ID NO:52)

### 3. Results and discussion

### Construction of vectors for replacing MHC regions

First, we constructed vectors necessary for a series of recombination operations. Considering the effect of translocational replacement on the expression of the surrounding genes, targeting vectors TV3R-dHLA and TV4-dH2 were designed to recombinantly introduce TV3R sequence or TV4 sequence between the MOG and GABBR1 loci, a relatively large stretch of intergenic sequence on the distal side from the human or mouse MHC region (Figure 5A, B). To increase the homologous recombination efficiency, CRISPR vectors PX458a-dHLACR1 and PX458a-dH2CR1 were generated to induce DNA double-strand breaks at the respective target sites.

TV3R encodes the loxP sequence required for translocation induction, the 3' sequence of the Neo-resistance gene for conferring G418 resistance upon the translocational recombination, the FRT sequence for removing the sequence in the targeting vector after the translocation induction, the hygromycin-resistance gene for drug selection upon targeting, and the red fluorescent gene mRuby2 for detecting the presence of the targeted chromosome.

Meanwhile, TV4 encodes the loxP sequence required for translocation induction, the 5' sequence of the Neo-resistance gene for conferring G418 resistance upon the translocational recombination, the FRT sequence for removing the sequence in the targeting vector after the translocation induction, the puromycin-resistance gene for drug selection upon targeting, and the green fluorescent gene EGFP for detecting the presence of the targeted chromosome. The TV3R and TV4 sequences are designed to leave the EGFP expression unit and the reconstituted Neo-resistance gene in the mouse chromosome 17 after the translocation induction, allowing sorting of the MHC-humanized mouse chromosome 17-positive cells using drugs and fluorescence.

Next, targeting vector TV-Tl 1/2-BS was generated to induce translocational recombination between the DAXX and KIFC1 loci, a relatively large stretch of intergenic sequence on the proximal side from the human and mouse MHC regions, and CRISPR vectors PX458.1a-pHLACR2 and PX458.1a-pH2CR1 were generated to cleave the respective target sites for increasing the recombination efficiency (Figure 5C). Since TV-Tl 1/2-BS contains blasticidin-resistance gene expression cassette, the recombinantly transfected cells were designed so that they can be selected by blasticidin resistance. In addition, the vector was designed to recombinantly introduce the blasticidin-resistance gene on the residual human chromosome 6, which will ultimately be removed from the ES cells. As a result, the effect on the expression of genes around the humanized MHC region is expected to be minimized because no foreign gene expression cassette is introduced on the proximal side from the MHC region on the MHC-humanized mouse chromosome 17.

### Generation of A9-MRC5 fused cell

In order to introduce human chromosome 6 of normal human fibroblast MRC5 cells into mouse ES cells (hereafter referred to as ES cells), it is necessary to generate fused cells of MRC5 cells and mouse fibroblast A9 cells with high chromosome donor ability. Specifically, to select the fused cells with a drug, neomycin-resistance gene was first introduced into MRC5 cells using lentivirus (Figure 4, Step 1 (encircled number 1; hereinafter, each of the subsequent steps will be numbered with a number in a circle as in step 1). Next, the neomycin-resistant MRC5 cells were fused with A9 cells, and the fused cell line hChA9, which had become resistant to both ouabain and G418, i.e., drugs that selectively kill human cells, was isolated (Figure 4, Step 2).

The presence of the sequence within the human HLA region in the isolated clones was confirmed by genomic PCR, and signals were detected in clones #1, 5, and 13 (Figure 6A). Furthermore, to confirm the presence of human chromosome 6 in hChA9#5, FISH analysis was performed using a probe specific to the human chromosome 6 centromere sequence, confirming the presence of two human chromosomes 6 in this fused cell clone (Figure 6B).

### Targeting of TV3R

The targeting vector TV3R-dHLA was recombinantly introduced into human chromosome 6 of hChA9#5 on the distal side from the MHC region to introduce the sequence necessary for the replacement of the MHC region (Figure 4, Step 3).

The resulting clones that became hygromycin-resistant were isolated and analyzed by genomic PCR, and a plurality of targeted cells TV3RhChA9 were isolated (Figure 7A). To exclude the possibility of mixed clones, TV3RhChA9#5-45a (128) was isolated by recloning from TV3RhChA9#5-45 (128) (Figure 7B). FISH analysis of TV3RhChA9#5-45a (128) confirmed that the majority of cells retained one human chromosome 6 (Figure 7C). Therefore, TV3RhChA9#5-45a (128) was used as a modified human chromosome 6 donor cell.

### Targeting of TV4

The distal side from the MHC region existing on chromosome 17 of B6-derived mouse ES cells RENKA was targeted with TV4-dH2 to introduce sequences necessary for swapping the MHC regions (Figure 4, Step 4).

Genomic PCR analysis of EGFP-positive clones confirmed a number of recombinantly transfected clones (Figure 8A). Since EGFP in the TV4 sequence can be used to confirm germline transmission if it is present only in the MHC-humanized allele, clone TV4RENKA#17 with 40 chromosomes, in which only one allele was targeted, was used in the subsequent experiments (Figure 8).

### Introduction of modified human chromosome 6

The modified human chromosome 6 of TV3RhChA9#5-45a (128) was introduced into TV4RENKA#17 (Figure 4, Step 5). Since ES cells usually have low chromosome transfer efficiency and fused cells tend to have lower chromosome donor ability than A9 cells, it is possible that the conventional chromosome transfer method using polyethylene glycol (PEG-MMCT method) cannot be used for the chromosome transfer. Therefore, a highly efficient chromosome transfer method (retro-MMCT method) using an ecotropic envelope protein derived from mouse leukemia virus was used to introduce the modified human chromosome 6.

ES cells that became hygromycin-resistant and mRuby2-positive were cloned, and chromosome preparations were made for karyotyping. While normal mouse cells have 40 chromosomes (XO has 39 chromosomes), TV3-4RENKA#17/5-45a-3 was found to have 41 chromosomes (XO had 40 chromosomes). In was also confirmed that the chromosome retained a submetacentric chromosome characteristic of human chromosome 6, and FISH analysis further revealed that this chromosome contained the human MHC region. Therefore, it was considered that the modified human chromosome 6 had been successfully introduced (Figure 9).

### Induction of chromosomal translocation using Cre/loxP

Recombinase Cre can induce translocation between loxP sequences on independent chromosomes. Accordingly, recombinase Cre was transiently expressed in TV3-4RENKA#17/5-45a-3 to induce translocation between the modified human chromosome 6 and the modified mouse chromosome 17, which were retained by TV3-4RENKA#17/5-45a-3, on the distal sides from the MHC regions (Figure 4, Step 6). Since induction of translocational recombination between loxPs present in the TV3 and TV4 sequences results in reconstitution of the Neo-resistance gene (Figure 10A), clones that became G418 resistant were isolated. Among these clones, the translocational recombination was found to have occurred in a clone with 41 chromosomes (40 chromosomes in XO) as confirmed by genomic PCR and FISH method. This clone was designated as "TV3-4RENKA Tl3/4#17-1" and used in the subsequent experiments (Figure 10B-D).

### Induction of chromosomal translocation using CRISPR/Cas9

Since translocation on the proximal side from the MHC region was planned to be induced using recombinase Dre as in the case on the distal side, ES cells were initially prepared by recombinantly introducing Dre recognition sequence rox into the proximal side. However, since translocation using Dre was inefficient and the expression of the HPRT gene, which was planned to be used as a selection marker, was highly auxotrophic in ES cells, it was found that the expression of the HPRT gene reconstituted by the recombinant translocation could not survive the selection culture with HAT. Therefore, the modification of mouse chromosome 17 was changed to the introduction of TV4 alone, and TV3-4RENKA Tl3/4#17-1 was generated without disrupting the Hprt gene in the ES cells.

Accordingly, translocation on the proximal side from the MHC region was induced using CRISPR/Cas9. Specifically, a CRISPR vector that cleaves the region extending from the KIFC1 gene to the DAXX gene in each of mouse chromosome 17 and human chromosome 6, and a translocation-inducing targeting vector TV-Tl1/2-BS were introduced into TV3-4RENKA Tl3/4#17-1, and blasticidin-resistant clones were isolated (Figure 4, Step 7). Among these clones, clone TV3-4RENKA Tlb#38 had 41 chromosomes (40 chromosomes in XO) and genomic PCR confirmed that the translocational recombination had occurred. In addition, FISH analysis showed that the modified human chromosome 6 had translocated with the modified chromosome 17, not with the wild-type chromosome 17, and thus TV3-4RENKA Tlb#38 was used in the subsequent experiments (Figure 11).

### Isolation of MHC-humanized ES cells

To ensure the undifferentiated nature of the MHC-humanized ES cells, it was initially planned to generate MHC-humanized ES cells by introducing the MHC-humanized mouse chromosome 17 generated thus far into low-passage ES cells and deleting the endogenous mouse chromosome 17 (Figure 4, Step 8'). However, this operation requires the generation of ES-A9 fused cells and the introduction of the MHC-humanized chromosome into ES cells, and it was found that chromosome abnormalities frequently occurred during these processes, which limited the operation. Therefore, an attempt was made to generate chimeric mice by allowing spontaneous elimination of the human chromosome from TV3-4RENKA Tlb#38 and isolating a number of MHC-humanized ES clones (Figure 4, Step 8).

Human chromosomes have been reported to be prone to elimination in mouse cells. Accordingly, TV3-4RENKA Tlb#38 was cultured for several days in the absence of selective agents to induce elimination of the residual human chromosome 6. To isolate the clones in which the residual human chromosome 6 had been eliminated, it was initially planned to use 6-thioguanine as a selective agent that can select for HPRT gene-deficient cells, but as mentioned above, it was found that the Hprt gene was difficult to use as a selective marker in ES cells.

Therefore, clones were isolated by sorting mRuby2-negative cells using the expression of mRuby2, i.e., a fluorescent gene designed to remain on the residual human chromosome 6, as an indicator. As a result, clone TV3-4RENKATlb#38dh6, in which the residual human chromosome 6 had been eliminated, was successfully isolated (Figure 12). Among these clones, TV3-4RENKA Tlbdh6#3 with 40 chromosomes (39 in XO) and the MHC-humanized mouse chromosome 17 confirmed by FISH method was used for the subsequent experiments. Whole genome sequencing analysis of TV3-4RENKA Tlbdh6#3 was also performed using the mouse genome sequence and the sequence in the human MHC region as references to identify the haplotype of the introduced human MHC gene group (Figure 12C).

### Establishment of MHC-humanized mice

Chimeric mice were generated from TV3-4RENKATlb#38dh6#3 and three mice with a coat color chimerism of 50%-60% were obtained (Figure 13A) (Figure 4, Step 9).

Cells derived from the ES cells with MHC-humanized chromosomes were designed to express EGFP (Figure 13B). Accordingly, the contribution rate of the MHC-humanized ES cells in the testis was analyzed using EGFP expression as an indicator, and it was found that almost 100% of the cells were EGFP-positive (Figure 13C). This suggested that intracytoplasmic sperm injection (ICSI/ROSI) of the testicular cells would have a high possibility of producing offsprings. Therefore, intracytoplasmic sperm injection was performed using the testicular cells from the chimeric mice (Figure 4, Step 10).

As a result, MHC-humanized mice whose entire bodies glowed green were successfully obtained by the germline transmission of the MHC-humanized ES cells (Figure 14A, B). Furthermore, genomic PCR also confirmed that the EGFP-positive individuals had the sequence of the human MHC region (Figure 14C). These results indicated that a MHC-humanized (heterozygous) mouse had been successfully established.

### Generation of MHC-humanized mice by natural breeding of chimeric mice

The previous results indicated that the MHC-humanized chimeric mice generated from B6-derived ES cells had low fertility. Therefore, humanization of MHC in XO ES cells (B6/CBA F1), which develop as females, was also performed in the same manner to generate chimeric mice (Figure 15A) (Figure 4, Step 9), which were crossed with wild-type mice.

As a result, MHC-humanized heterozygous mice were successfully obtained by natural breeding (Figure 15B-D) (Figure 4, Step 10).

### 4. Analysis of expression of human MHC genes in MHC-humanized (heterozygous) mice

### <Method>

### Analysis of expression of human MHC (also called HLA) proteins

To prepare splenocytes, an excised spleen was first chopped into small pieces with a knife, and then the tissue pieces were ground using the frosted portion of a glass slide. Cells were then lysed with 0.83% ammonium chloride and passed through nylon mesh to prepare splenocytes. For flow cytometric analysis, cells were stained with Pacific Blue-labeled anti-mouse CD19 antibody (Biolegend), anti-HLA class I antibody (MBL) labeled with APC using APC Conjugation Kit-Lightning-Link (Abcam) or APC-labeled anti-HLA-DR,DP,DQ antibody (Biolegend), and analyzed using FACS AriaIII (BD). In Western blot analysis, detection was performed using anti-HLA-A,B,C antibody (MBL), anti-HLA-A antibody (Abcam), anti-HLA-DRA antibody (Abcam) or HRP-labeled anti-ACTB antibody (Genscript).

To prepare lung-derived fibroblasts, excised lung tissue was first chopped into small pieces with a knife, treated with 0.1% collagenase A (Roche) for 1 hour, the cells were released by pipetting, and then passed through nylon mesh to prepare lung-derived fibroblasts. The lung-derived fibroblasts were cultured in 10% FCS-supplemented DMEM.

### Results

Expression of human MHC gene in splenocytes was analyzed at the protein level. First, splenocytes were prepared from the MHC-humanized (heterozygous) mice (Het) established from wild-type (Wt) and XO ES cells, and expression of human MHC proteins was analyzed by Western blotting using anti-HLA-A,B,C antibody, anti-HLA-A antibody, and anti-HLA-DRA antibody. As a result, expression of human MHC was specifically detected in Het-derived splenocytes (Figure 16A).

Next, an analysis was performed for the expression of HLA proteins on the cell surface. Splenocytes are mainly composed of B cells and T cells, and MHC class I should be found to be expressed in all cells and MHC class II in B cells. Therefore, flow cytometric analysis was performed using an antibody against CD19 as a B cell marker, anti-HLA class I antibody that recognizes human MHC class I, and anti-HLA-DP,DQ,DR antibody that recognizes human MHC class II, and found that HLA class I was detected in all Het-derived splenocytes, and HLA-DP,DQ,DR were specifically expressed in Het-derived B cells (Figure 16B).

### [SEQUENCE LISTING FREE TEXT]

SEQ ID NO:1: Synthetic DNA
   Other information:
   /label = "EF1" (Location: 30..1217)
   /label = "Hyg" (Location: 1230..2267)
   /label = "RGpA" (Location: 2274..2808)
   complement/label = "Origin pMB1 ori (pUC)" (Location: 3562..4176)
   complement/label = "Bacterial Selection Amp" (Location: 4336..5196)
SEQ ID NO:2: Synthetic DNA
   Other information:
   /label = "FRT" (Location: 18..65)
   complement/label = "BGHpA" (Location: 66..342)
   complement/label = "3'Neo" (Location: 347..805)
   /label = "SA" (Location: 806..871)
   complement/label = "loxP" (Location: 872..905)
SEQ ID NOS:3-8: Synthetic DNAs
SEQ ID NO:9: Synthetic DNA
   Other information:
   /lebel = "P2A"/note = "P2A" (Location: 46..111)
   /lebel = "mRuby2" (Location: 118..831)
SEQ ID NOS:10-11: Synthetic DNAs
SEQ ID NO:12: Synthetic DNA
   Other information:
   /label = "EF1" (Location: 30..1217)
   /label = "EGFP" (Location: 1230..1949)
   /label = "RGpA" (Location: 1956..2490)
   complement/label = "Origin pMB1 ori (pUC)" (Location: 3244..3858)
   /label = "Bacterial Selection Amp" (Location: 4018..4878)
SEQ ID NO:13: Synthetic DNA
   Other information:
   complement/label = "Puro" (Location: 22..618)
   complement /label = "T2A" (Location: 619..681)
   /label = "SA" (Location: 684..750)
   complement/label = "loxP" (Location: 751..784)
   /label = "SD" (Location: 788..854)
   complement/label = "5'Neo" (Location: 855..1199)
   complement /label = "P2A" (Location: 1200..1265)
SEQ ID NOS:14-21: Synthetic DNAs
SEQ ID NO:22: Synthetic DNA
   Other information:
   /label = "Amp" (Location: 819..1679)
   /label = "Origin pMB1 ori (pUC)" (Location: 1839..2453)
   /label = "LA" (Location: 2957..4041)
   /label = "CAG promoter" (Location: 4047..5746)
   /label = "Hprt" (Location: 5756..6412)
   /label = "RGpA" (Location: 6439..6968)
   /label = "RA" (Location: 6975..8363)
SEQ ID NO:23: Synthetic DNA
   Other information:
   /label = "Blasticidin-resistance gene" (Location: 126..524)
SEQ ID NO:24: Synthetic DNA
   Other information:
   /label = "U6 promoter" (Location: 1..249)
   /label = "chimeric guide RNA scaffold" (Location: 268..343)
   /label = "U6 promoter" (Location: 344..349)
   /label = "CBh" (Location: 440..1238)
   /label = "3xFLAG" (Location: 1251..1319)
   /label = "NLS(1)" (Location: 1320..1370)
   /label = "hSpCsn1" (Location: 1371..5471)
   /label = "NLS" (Location: 5472..5519)
   /label = "T2A" (Location: 5526..5588)
   /label = "Ametrine" (Location: 5589..6305)
   /label = "BGHpA" (Location: 6315..6546)
   /label = "Bacterial Selection Amp" (Location: 7612..8472)
   /label = "Origin pMB1 ori (pUC)" (Location: 8632..9246)
SEQ ID NO:27: Synthetic DNA
   Other information:
   /label = "U6 promoter" (Location: 1..249)
   /label = "chimeric guide RNA scaffold" (Location: 268..353)
   /label = "U6 promoter" (Location: 354..359)
   /label = "CBh" (Location: 450..1248)
   /label = "3xFLAG" (Location: 1261..1329)
   /label = "NLS(1)" (Location: 1330..1380)
   /label = "hSpCsn1" (Location: 1381..5481)
   /label = "NLS" (Location: 5482..5529)
   /label = "T2A" (Location: 5536..5598)
   /label = "Ametrine" (Location: 5599..6315)
   /label = "BGHpA" (Location: 6325..6556)
   /label = "Bacterial Selection Amp" (Location: 7622..8482)
   /label = "Origine pMB1 ori (pUC)" (Location: 8642..9256)
   SEQ ID NOS:28-52: Synthetic DNAs

## Claims

1. A vector set comprising a combination of the following targeting vectors (A), (B) and (D), or a combination of the following targeting vectors (C) and (D):
(A) a targeting vector 1 that is incorporated into human chromosome 6 at an arbitrary position P1 located on the distal side from a major histocompatibility complex (human MHC) region on the short arm of the human chromosome 6,
wherein the targeting vector 1 causes translocational recombination between a region extending from the aforementioned P1 to the distal end and a region on a non-human mammalian chromosome, the region extending from position p1, which corresponds to the aforementioned P1 and is located on the distal side from a MHC (non-human MHC) region existing on the non-human mammalian chromosome, to the distal end, and the targeting vector 1 also contains a gene cassette 1 containing a recombinase recognition sequence, one partial sequence of a drug resistance gene 1 for drug selection, which is reconstituted upon the translocational recombination, and a drug resistance gene 2 for drug selection after targeting;
(B) a targeting vector 2 that is incorporated into the non-human mammalian chromosome in which the MHC region exists, at position p1 that corresponds to the aforementioned P1 and that is located on the distal side from the MHC (non-human MHC) region,
wherein the targeting vector 2 causes translocational recombination between the region extending from the aforementioned p1 to the distal end and the region extending from the aforementioned P1 to the distal end on the human chromosome 6, and the targeting vector 2 also contains a gene cassette 2 containing a recombinase recognition sequence, the other sequence of the drug resistance gene 1, and a drug resistance gene 3 for drug selection after targeting;
(C) a targeting vector 3 that causes translocational recombination between a region on human chromosome 6, the region extending from an arbitrary position P1, which is located on the distal side from a human MHC region and targeted for cleavage by a genome editing tool, to the distal end, and a region on a non-human mammalian chromosome in which MHC exists, the region extending from position p1, which corresponds to the aforementioned P1 and is located on the distal side from the non-human MHC region, to the distal end,
wherein the targeting vector 3 contains a homologous sequence of the sequence of partial region R1a located between the aforementioned P1 and the human MHC region, a drug resistance gene 4 for drug selection after targeting, and a homologous sequence of the sequence of partial region R2b located on the distal side from the aforementioned p1, or the targeting vector 3 contains a homologous sequence of the sequence of partial region R1b located on the distal side from the aforementioned P1, a drug resistance gene 4 for drug selection after targeting, and a homologous sequence of the sequence of partial region R2a located between the aforementioned p1 and the non-human MHC region; and
(D) a targeting vector 4 that causes translocational recombination between a region extending from an arbitrary position P2, which is located on the proximal side from the human MHC region and targeted for cleavage by a genome editing tool, to the distal end and a region on the non-human mammalian chromosome in which the non-human MHC exists, the region extending from position p2, which corresponds to the aforementioned P2 and located on the proximal side from the non-human MHC region, to the distal end,
wherein the targeting vector 4 contains a homologous sequence of the sequence of partial region R3a located on the proximal side from the aforementioned P2, a drug resistance gene 5 for drug selection after targeting, and a homologous sequence of the sequence of partial region R4b located between the aforementioned p2 and the non-human MHC region, or the targeting vector 4 contains a homologous sequence of the sequence of partial region R3b located between the aforementioned p2 and the human MHC region, a drug resistance gene 5 for drug selection after targeting, and a homologous sequence of the sequence of partial region R4a located on the proximal side from the aforementioned p2.

2. The vector set according to claim 1, further comprising the following vectors (E) and (F):
(E) a genome editing tool vector 1 that cleaves human chromosome 6 at the arbitrary position P1; and
(F) a genome editing tool vector 2 that cleaves the non-human chromosome at the arbitrary position p1.

3. The vector set according to claim 1, further comprising the following vectors (G) and (H):
(G) a genome editing tool vector 3 that cleaves human chromosome 6 at the arbitrary position P2; and
(H) a genome editing tool vector 4 that cleaves the non-human chromosome at the arbitrary position p2.

4. The vector set according to any one of claims 1-3, wherein the human MHC region comprises a class I region, a class II region, a class III region, or a combination of these regions.

5. The vector set according to claim 4, wherein the class I region comprises at least one selected from the group consisting of HLA-A, HLA-B, HLA-C, HLA-E, HLA-F, HLA-G, MICA, and MICB.

6. The vector set according to claim 4, wherein the class II region comprises at least one selected from the group consisting of HLA-DP, HLA-DQ, HLA-DR, HLA-DM, and HLA-DO.

7. The vector set according to claim 4, wherein the region comprising the class I region is a region containing HLA-A, HLA-B, HLA-C, HLA-E, HLA-F, HLA-G, MICA, and MICB.

8. The vector set according to claim 4, wherein the region comprising the class II region is a region containing HLA-DP, HLA-DQ, HLA-DR, HLA-DM, and HLA-DO.

9. The vector set according to claim 4 in which the region comprising both class I and class II regions is a region between the DAXX locus and the MOG locus.

10. The vector set according to any one of claims 1-3, wherein the arbitrary position P1 is a position between the MOG locus and the GABBR1 locus.

11. The vector set according to any one of claims 1-3, wherein the arbitrary position P2 is a position between the KIFC1 locus and the DAXX locus.

12. The vector set according to any one of claims 1-3, wherein the non-human mammal is a rodent.

13. The vector set according to claim 12, wherein the rodent is a mouse.

14. The vector set according to claim 13, wherein the arbitrary position p1 is a position between the Mog locus and the Gabbr1 locus.

15. The vector set according to claim 13, wherein the arbitrary position p2 is a position between the Kifc1 locus and the Daxx locus.

16. The vector set according to any one of claims 1-3, wherein the drug resistance gene 1 is neomycin-resistance gene, the drug resistance gene 2 is hygromycin-resistance gene, and/or the drug resistance gene 3 is puromycin-resistance gene.

17. The vector set according to any one of claims 1-3, wherein the drug resistance gene 4 is neomycin-resistance gene and/or the drug resistance gene 5 is blasticidin-resistance gene.

18. The vector set according to any one of claims 1-3, wherein, in the gene cassette 1, the recombinase recognition sequence is loxP, the drug resistance gene 1 is neomycin-resistance gene, and the drug resistance gene 2 is hygromycin-resistance gene.

19. The vector set according to any one of claims 1-3, wherein the gene cassette 1 comprises a construct represented by the following formula (1):
BGHpA-3'Neo-SA-loxP-EF1-Hyg-P2A-mRuby2-RGpA (1)
(wherein, BGHpA represents a bovine growth hormone-derived poly A addition signal sequence, 3'Neo represents a part of the 3' sequence of neomycin-resistance gene, SA represents a splicing acceptor sequence, loxP represents a recombinase recognition sequence, EF1 represents human elongation factor 1α-derived promoter sequence, Hyg represents hygromycin-resistance gene, P2A represents porcine teschovirus-derived 2A sequence, mRuby2 represents red fluorescent gene, and RGpA represents rabbit β-globin-derived poly A addition signal sequence).

20. The vector set according to any one of claims 1-3, wherein, in the gene cassette 2, the recombinase recognition sequence is loxP, the drug resistance gene 1 is neomycin-resistance gene, and the drug resistance gene 3 is puromycin-resistance gene.

21. The vector set according to any one of claims 1-3, wherein the gene cassette 2 comprises a construct represented by the following formula (2):
EF1-EGFP-P2A-5'Neo-SD-loxP-SA-T2A-Puro-RGpA (2)
(wherein, EF1 represents human elongation factor 1α-derived promoter sequence, EGFP represents green fluorescent gene, P2A represents porcine teschovirus-derived A sequence, 5'Neo represents a part of the 5' sequence of neomycin-resistance gene, SD represents a splicing donor sequence, loxP represents a recombinase recognition sequence, SA represents a splicing acceptor sequence, T2A represents Thosea asigna virus-derived 2A sequence, Puro represents puromycin-resistance gene, and RGpA represents rabbit β-globin-derived poly A addition signal sequence).

22. A non-human mammalian cell with a humanized MHC region, the comprising a chromosome in which a MHC region on the non-human mammalian chromosome has been replaced with a MHC region on human chromosome by the vector set according to any one of claims 1-21.

23. The cell according to claim 22, wherein the cell is an ES cell.

24. A non-human mammal, which is generated from the cell according to claim 22 and in which the MHC region has been humanized.

25. The non-human mammal according to claim 24, wherein the non-human mammal is a rodent.

26. The non-human mammal according to claim 25, wherein the rodent is a mouse.

27. The non-human mammal according to claim 26, comprising a chromosome in which a chromosome portion extending at least from the Daxx locus to the Mog locus on mouse chromosome 17 has been replaced with a chromosome portion extending at least from the DAXX locus to the MOG locus on human chromosome 6.

28. A method for producing a non-human mammal with a humanized MHC region, the method comprising the steps of:
(1) generating a cell comprising a chromosome in which a MHC region on a non-human mammalian chromosome has been replaced with a MHC region on human chromosome using the vector set according to any one of claims 1-3; and
(2) creating a non-human mammal with a humanized MHC region from the cell obtained in (1) above.

29. The method according to claim 28, wherein the non-human mammal is a rodent.

30. The method according to claim 29, wherein the rodent is a mouse.

31. A method for producing a non-human mammal with a humanized MHC region, the method comprising the step of transplanting the cell according to claim 22 into a foster parent of a non-human mammal to create a non-human mammal having a chromosome with a humanized MHC region.

32. The method according to claim 31, wherein the non-human mammal is a rodent.

33. The method according to claim 32, wherein the rodent is a mouse.
